# EUROPEAN PATENT APPLICATION

(11) **EP 3 552 614 A1**
(43) Date of publication of application: **16.10.2019**
(21) Application number: 17878182.9
(22) Date of filing: 08.12.2017
(51) Int. Cl.: A61K 35/761, A61K 31/7068, A61K 39/395, A61K 45/06

(54) **ANTICANCER COMPOSITION COMPRISING RECOMBINANT ADENOVIRUS EXPRESSING DEGRADATION FACTOR FOR EXTRACELLULAR MATRIX**

(30) Priority: 09.12.2016 KR 20160167265
(71) Applicant: Genemedicine Co., Ltd., Seongdong-gu Seoul 04763 (KR)
(72) Inventor: YUN, Chae Ok, Seoul 06583 (KR); AHN, Hyo Min, Incheon 22730 (KR)
(74) Representative: Schiweck Weinzierl Koch Patentanwälte Partnerschaft mbB
(86) International application number: PCT/KR2017/014409
(87) International publication number: WO 2018/106068

(57) **Abstract**

The present invention relates to an anticancer composition comprising a recombinant adenovirus which expresses degradation factors for the extracellular matrix. The recombinant adenovirus according to the present invention exhibits an excellent anti-tumor effect by remarkably reducing the main structural components of the extracellular matrix in a tumor tissue, including collagen I, collagen III, fibronectin, elastin, and the like and highly expressing a therapeutic gene selectively only in tumor cells through viral proliferation. Particularly, when administered in combination with therapeutic materials, such as anticancer agents or immune checkpoint inhibitors, the recombinant adenovirus significantly increases the diffusion and distribution of the co-administered therapeutic materials in tumor tissues while allowing the exertion of the preexisting anticancer effects, thereby further improving an anti-cancer effect. Accordingly, the present invention may be available as a core technique in the cancer treatment field.

<110>    Industry-university Cooperation Foundation Hanyang University

<120>    Anti-tumor composition comprising oncolytic recombinant
         adenovirus  expressing extracellular matrix-degrading factor

<130>    O20170245WO_X17U10C0241

<150>    KR 10-2016-0167265
<151>    2016-12-09

<160>    5

<170>    KoPatentIn 3.0

<210>    1
<211>    648
<212>    DNA
<213>    IL-12A

<400>    1
atgtgtcaat cacgctacct cctctttttg gccacccttg ccctcctaaa ccacctcagt        60

ttggccaggg tcattccagt ctctggacct gccaggtgtc ttagccagtc ccgaaacctg       120

ctgaagacca cagatgacat ggtgaagacg gccagagaaa aactgaaaca ttattcctgc       180

actgctgaag acatcgatca tgaagacatc acacgggacc aaaccagcac attgaagacc       240

tgtttaccac tggaactaca caagaacgag agttgcctgg ctactagaga gacttcttcc       300

acaacaagag ggagctgcct gcccccacag aagacgtctt tgatgatgac cctgtgcctt       360

ggtagcatct atgaggactt gaagatgtac cagacagagt tccaggccat caacgcagca       420

cttcagaatc acaaccatca gcagatcatt ctagacaagg gcatgctggt ggccatcgat       480

gagctgatgc agtctctgaa tcataatggc gagactctgc gccagaaacc tcctgtggga       540

gaagcagacc cttacagagt gaaaatgaag ctctgcatcc tgcttcacgc cttcagcacc       600

cgcgtcgtga ccatcaacag ggtgatgggc tatctgagct ccgcctga                    648


<210>    2
<211>    1008
<212>    DNA
<213>    IL-12B

<400>    2
atgtgtcctc agaagctaac catctcctgg tttgccatcg ttttgctggt gtctccactc        60

atggccatgt gggagctgga gaaagacgtt tatgttgtag aggtggactg gactcccgat       120

gcccctggag aaacagtgaa cctcacctgt gacacgcctg aagaagatga catcacctgg       180

acctcagacc agagacatgg agtcataggc tctggaaaga ccctgaccat cactgtcaaa       240

gagtttctag atgctggcca gtacacctgc cacaaaggag gcgagactct gagccactca       300

catctgctgc tccacaagaa ggaaaatgga atttggtcca ctgaaatttt aaaaaatttc       360

aaaaacaaga ctttcctgaa gtgtgaagca ccaaattact ccggacggtt cacgtgctca       420

tggctggtgc aaagaaacat ggacttgaag ttcaacatca agagcagtag cagttcccct 480

gactctcggg cagtgacatg tggaatggcg tctctgtctg cagagaaggt cacactggac 540

caaagggact atgagaagta ttcagtgtcc tgccaggagg atgtcacctg cccaactgcc 600

gaggagaccc tgcccattga actggcgttg gaagcacggc agcagaataa atatgagaac 660

tacagcacca gcttcttcat cagggacatc atcaaaccag acccgcccaa gaacttgcag 720

atgaagcctt tgaagaactc acaggtggag gtcagctggg agtaccctga ctcctggagc 780

actccccatt cctacttctc cctcaagttc tttgttcgaa tccagcgcaa gaaagaaaag 840

atgaaggaga cagaggaggg gtgtaaccag aaaggtgcgt tcctcgtaga gaagacatct 900

accgaagtcc aatgcaaagg cgggaatgtc tgcgtgcaag ctcaggatcg ctattacaat 960

tcctcatgca gcaagtgggc atgtgttccc tgcagggtcc gatcctag 1008

<210> 3
<211> 597
<212> DNA
<213> IRES

<400> 3
tagcgatccg cccctctccc tccccccccc ctaacgttac tggccgaagc cgcttggaat 60

aaggccggtg tgcgtttgtc tatatgttat tttccaccat attgccgtct tttggcaatg 120

tgagggcccg gaaacctggc cctgtcttct tgacgagcat tcctaggggt ctttcccctc 180

tcgccaaagg aatgcaaggt ctgttgaatg tcgtgaagga agcagttcct ctggaagctt 240

cttgaagaca aacaacgtct gtagcgaccc tttgcaggca gcggaacccc ccacctggcg 300

acaggtgcct ctgcggccaa aagccacgtg tataagatac acctgcaaag gcggcacaac 360

cccagtgcca cgttgtgagt tggatagttg tggaaagagt caaatggctc tcctcaagcg 420

tattcaacaa ggggctgaag gatgcccaga aggtacccca ttgtatggga tctgatctgg 480

ggcctcggta cacatgcttt acatgtgttt agtcgaggtt aaaaaaacgt ctaggccccc 540

gaaccacggg gacgtggttt tccttttgaa aaacacgatg ataatatggc cacaacc 597

<210> 4
<211> 1080
<212> DNA
<213> Decorin

<400> 4
atgaaggcca ctatcatcct ccttctgctt gcacaagttt cctgggctgg accgtttcaa 60

cagagaggct tatttgactt tatgctagaa gatgaggctt ctgggatagg cccagaagtt 120

cctgatgacc gcgacttcga gccctcccta ggcccagtgt gccccttccg ctgtcaatgc 180

catcttcgag tggtccagtg ttctgatttg ggtctggaca aagtgccaaa ggatcttccc 240

cctgacacaa ctctgctaga cctgcaaaac aacaaaataa ccgaaatcaa agatggagac 300

tttaagaacc tgaagaacct tcacgcattg attcttgtca acaataaaat tagcaaagtt 360

agtcctggag catttacacc tttggtgaag ttggaacgac tttatctgtc caagaatcag 420

ctgaaggaat tgccagaaaa aatgcccaaa actcttcagg agctgcgtgc ccatgagaat 480

gagatcacca aagtgcgaaa agttactttc aatggactga accagatgat tgtcatagaa 540

ctgggcacca atccgctgaa gagctcagga attgaaaatg gggctttcca gggaatgaag 600

aagctctcct acatccgcat tgctgatacc aatatcacca gcattcctca aggtcttcct 660

ccttccctta cggaattaca tcttgatggc aacaaaatca gcagagttga tgcagctagc 720

ctgaaaggac tgaataattt ggctaagttg ggattgagtt tcaacagcat ctctgctgtt 780

gacaatggct ctctggccaa cacgcctcat ctgagggagc ttcacttgga caacaacaag 840

cttaccagag tacctggtgg gctggcagag cataagtaca tccaggttgt ctaccttcat 900

aacaacaata tctctgtagt tggatcaagt gacttctgcc cacctggaca caacaccaaa 960

aaggcttctt attcgggtgt gagtcttttc agcaacccgg tccagtactg ggagatacag 1020

ccatccacct tcagatgtgt ctacgtgcgc tctgccattc aactcggaaa ctataagtaa 1080

1080

<210> 5
<211> 558
<212> DNA
<213> Relaxin

<400> 5
atgcctcgcc tgttcttgtt ccacctgcta gaattctgtt tactactgaa ccaattttcc 60

agagcagtcg cggccaaatg gaaggacgat gttattaaat tatgcggccg cgaattagtt 120

cgcgcgcaga ttgccatttg cggcatgagc acctggagca aaaggtctct gagccaggaa 180

gatgctcctc agacacctag accagtggca gaaattgtac catccttcat caacaaagat 240

acagaaacta taattatcat gttggaattc attgctaatt tgccaccgga gctgaaggca 300

gccctatctg agaggcaacc atcattacca gagctacagc agtatgtacc tgcattaaag 360

gattccaatc ttagctttga agaatttaag aaacttattc gcaataggca aagtgaagcc 420

gcagacagca atccttcaga attaaaatac ttaggcttgg atactcattc tcaaaaaaag 480

agacgaccct acgtggcact gtttgagaaa tgttgcctaa ttggttgtac caaaaggtct 540

cttgctaaat attgctga 558

## Description

### [Technical Field]

The present invention relates to an anticancer composition comprising a recombinant adenovirus which expresses degradation factors for the extracellular matrix.

The present invention was conducted as Project No. 20160000002116 under the support of the Korean Ministry of Science, ICT and Future Planning, and the research management agency of the above project is the National Research Foundation of Korea, the name of the research business is Basic Research Business of Science and Engineering Field/Veteran Researcher Supporting Business/Acceleration Research (Strategic-Follow-Up Research Support), and the name of the research project is Development of Nanomaterial Hybrid Gene Carrier for Selective Tumor Control. The present invention was conducted as Project No. 20160000002536 under the support of the Korean Ministry of Science, ICT and Future Planning, and the research management agency of the above project is the National Research Foundation of Korea, the name of the research business is Source Technology Development Business/Biomedical Technology Development Business/Technology Development Business Based on Next-Generation New Drug, and the name of the research project is Study on Development and Practical Application of Tumor-Killing

Adenovirus Candidate Material for Treatment of Pancreatic Cancer.

The present patent application claims priority to and the benefit of Korean Patent Application No. 10-2016-0167265, filed on December 9, 2016, the disclosure of which is incorporated herein by reference in its entirety.

### [Background Art]

Cancer is a disease that is the leading cause of disease death worldwide, and is an intractable disease of which 50% or more of patients die in spite of a complex treatment such as surgery, radiation treatment, and chemotherapy (WHO: World Health Report, 2001). Cancer treatment was developed in the order of surgery, radiation treatment, and anticancer agent treatment. Surgery is effective for early-stage cancer, but most cancers that have metastasized need a combination of radiation treatment or anticancer treatment besides surgery. In particular, since these treatment methods have significant side effects on normal cells and have a low cure rate due to resistance to multiple drugs of cancer cells, there is an urgent need for developing a new anticancer treatment method. In particular, the development of a therapeutic agent for refractory (resistant) cancer associated with recurrence or metastasis of cancer is urgently needed. Due to such a practical necessity, among a total of 2,356 gene therapy clinical trials based on the year 2016, about 64.4% of the clinical trials were conducted for cancer as a target disease, and the clinical trial with the highest frequency has been conducted among various disorders.

As described above, examples of standard treatment methods currently used for cancer include surgery, radiation treatment, and anticancer agent treatment. In the early stage, that is, when cancer has metastasized only to a local or peripheral lymph gland, the cancer can be completely cured by only surgery, but when the metastasis thereof further progresses, there is a limitation in treatment, so that only a few patients with cancer can be cured through surgery. Accordingly, most cancers should be treated in combination with radiation and anticancer agent treatment in addition to surgery. However, since both radiation and anticancer agent treatment are not a targeted treatment method, they cannot distinguish cancer cells to be treated from normal cells, so that some normal cells are also damaged during treatment. Among normal cells, cells which are rapidly divided and proliferated, that is, blood cells formed in bone marrow, epithelial cells of the gastrointestinal tract including the oral cavity, hair cells, germ cells producing sperm and eggs, and the like are significantly affected. Since the side effects on normal cells are large, the cure rate for actual tumors is extremely low.

An ideal anticancer agent should be able to remove cancer cells without damaging normal cells. Unfortunately, no drug developed so far satisfies the conditions described above, and toxicity is merely compared with effects for preferred treatment. When the chemotherapeutic agents that have been intensively developed recently are classified, the first is a differentiation derivative capable of blocking the maturation of tumor cells by eliminating the proliferation ability of neocytes or allowing neocytes to be cells at telophase; the second is an anti-metastasis drug capable of disrupting the ability to invade and metastasize by altering surface characteristics of malignant tumor cells; the third is a hypoxic hepatocytic tumor-specific drug which induces a reduction reaction from the oxygen deficiency state in solid cancer cells; the fourth is a tumor cell-specific radiation drug, and the like.

New therapeutic materials as described above have enhanced anticancer effects as compared to preexisting anticancer therapies. In particular, examples of a drug showing better potential include taxane derivatives (for example; docetaxel), camptothecin derivatives, thymidylate synthase inhibitors (for example; raltitrexed), nucleoside derivatives (gemcitabine), 5-FU oral derivatives, and the like. However, the toxicity of these chemotherapeutic agents still remains a problem to be solved.

Recently, as many molecular biological characteristics of cancer have been elucidated, targeted therapeutic agents that attack only specific cancer cells have been developed. Targeted cancer treatment refers to the use of drugs that block cancer from growing and proliferating by interfering with the activity of special molecules involved in the growth and onset of cancer. When focused on the change in molecules and cells, targeted treatment has an advantage in that side effects can be minimized because targeted treatment selectively attacks only cancer cells while relatively minimizing damage to normal cells. Target therapeutic agents were made so as to target molecules that are characteristics of most cancer cells and exhibit the effects thereof. As the molecular target, molecules involved in the signal transduction pathway, angiogenesis, matrix, cell cycle regulation, apoptosis, and the like of cancer cells have been used, but additional studies are needed until their clinical application.

Further, as a new approach for the treatment of cancer, studies on immunotherapy using tumor-specific immune activity of the body have been conducted. However, it is difficult to perform the immune treatment because cancer has the ability not only to cleverly evade and destroy various host immune responses, and as a result sustainably maintains the survival of tumors by inducing the immunosuppression tumor microenvironment, but also to escape from the activated anti-tumor immune response even though the immune system is activated. Accordingly, in order to enhance the immune response against cancer cells by improving the immunosuppression tumor microenvironment, studies using cytokine genes such as IL-12, IL-18, interferon-gamma (IFN-γ), a granulocyte-macrophage colony-stimulating factor (GM-CSF), and a tumor necrosis factor-alpha (TNF-α), costimulatory factors such as B7 molecules, dendritic cells (DCs) directly serving as antigen presenting cells (APCs), T cells activated by tumor antigens, natural killer cells (NKCs), and the like have been carried out in various directions. In particular, IL-12 is usually secreted from APCs such as monocytes, macrophages, and DCs, and is known to directly act on cytotoxic T lymphocyctes (CTLs) and NK cells capable of effectively removing cancer cells to activate these cells, induce the secretion of IFN-γ, and enhance the ability to kill cancer cells. In addition, IL-12 plays an important role in promoting differentiation into T helper 1 (TH1) cells by acting on naive CD4⁺ lymphocytes, and as a result, activating an anticancer immune response by inducing and enhancing the cell-mediated immune response which plays a pivotal role in the anticancer immune response.

Based on this technical background, the present inventors reported an anti-tumor effect of YKL-1 (Ad-E1B-55k) as an E1B-55k gene-deleted tumor-selective killing adenovirus, and also reported an anti-tumor effect of GM-CSF using an RdB adenovirus with a tumor-selective killing ability more enhanced than YKL-1, due to the deletion of the E1B gene and a modification in an Rb binding site of E1A (Korean Patent Application Laid-Open No. 10-2012-0010697). However, although the immunosuppression tumor microenvironment is improved, there are still many limitations in completely curing cancer due to the low immunogenicity of tumors.

### [Disclosure]

### [Technical Problem]

As a result of intensive research on developing a new treatment method capable of improving an anti-cancer effect, the present inventors prepared a tumor-selective killing recombinant adenovirus which expresses degradation factors for the extracellular matrix, and overcame the limitation of preexisting anticancer agent treatments and simultaneously exhibited a very remarkable anti-tumor effect through a combination of the recombinant adenovirus with preexisting anticancer agents or immune checkpoint inhibitors, thereby completing the present invention based on this.

Accordingly, an object of the present invention is to provide a pharmaceutical composition for aiding an anticancer agent, comprising a recombinant adenovirus which expresses degradation factors for the extracellular matrix.

Another object of the present invention is to provide a pharmaceutical composition for treating cancer, administered in combination with immune checkpoint inhibitors and comprising a recombinant adenovirus which expresses degradation factors for the extracellular matrix.

However, technical problems to be achieved by the present invention are not limited to the aforementioned problems, and other problems that are not mentioned may be clearly understood by those skilled in the art from the following description.

### [Technical Solution]

In order to achieve the objects of the present invention as described above, provided is a pharmaceutical composition for aiding an anticancer agent comprising a recombinant adenovirus comprising a gene encoding Interleukin 12 (IL-12); and a gene encoding decorin or relaxin.

As an embodiment of the present invention, the recombinant adenovirus may be with the E1 and/or E3 region deleted.

As another embodiment of the present invention, the gene encoding IL-12 may be inserted into the E1 region of the recombinant adenovirus, and the gene encoding decorin or relaxin may be inserted into the E1 or E3 region of the adenovirus.

As still another embodiment of the present invention, the composition may be administered simultaneously, separately, or sequentially in combination with immune checkpoint inhibitors, and the immune checkpoint inhibitors may be any one selected from the group consisting of PD-1 antagonists, PD-L1 antagonists, CTLA-4 antagonists, PD-L2 antagonists, CD27 antagonists, CD28 antagonists, CD70 antagonists, CD80 antagonists, CD86 antagonists, CD137 antagonists, CD276 antagonists, KIRs antagonists, LAG3 antagonists, TNFRSF4 antagonists, GITR antagonists, GITRL antagonists, 4-1BBL antagonists, and a combination thereof.

As yet another embodiment of the present invention, the cancer may be any one selected from the group consisting of gastric cancer, lung cancer, non-small cell lung cancer, breast cancer, ovarian cancer, liver cancer, bronchial cancer, nasopharyngeal cancer, laryngeal cancer, pancreatic cancer, bladder cancer, colon cancer, cervical cancer, bone cancer, non-small cell bone cancer, hematologic malignancy, skin cancer, head or neck cancer, uterine cancer, colorectal cancer, anal near cancer, colon cancer, fallopian tube cancer, endometrial cancer, vaginal cancer, vulva cancer, Hodgkin's disease, esophageal cancer, small intestine cancer, endocrine cancer, thyroid cancer, parathyroid cancer, adrenal cancer, soft tissue sarcoma, urethral cancer, penile cancer, prostate cancer, chronic or acute leukemia, lymphocytic lymphoma, kidney or hydroureter cancer, renal cell carcinoma, renal pelvic carcinoma, salivary gland cancer, sarcoma cancer, pseudomyxoma peritonei, hepatoblastoma, testicular cancer, glioblastoma, cheilocarcinoma, ovarian germ cell tumors, basal cell carcinoma, multiple myeloma, gallbladder cancer, choroidal melanoma, cancer of the ampulla of Vater, peritoneal cancer, tongue cancer, small cell cancer, pediatric lymphoma, neuroblastoma, duodenal cancer, ureteral cancer, astrocytoma, meningioma, renal pelvis cancer, pudendum cancer, thymus cancer, central nervous system tumors, primary central nervous system lymphoma, spinal cord tumors, brain stem neuroglioma, and pituitary adenoma, and may be a recurrent cancer or an anticancer agent-resistant cancer.

As yet another embodiment of the present invention, the composition may enhance anti-tumor immunity.

Further, the present invention provides a method for enhancing the therapeutic efficacy of anticancer agents comprising: administering to an individual a recombinant adenovirus comprising a gene encoding IL-12; and a gene encoding decorin or relaxin .

In addition, the present invention provides a method for treating cancer comprising administering to an individual a recombinant adenovirus comprising a gene encoding IL-12; and a gene encoding decorin or relaxin .

### [Advantageous Effects]

The recombinant adenovirus according to the present invention exhibits an excellent anti-tumor effect by remarkably reducing the main structural components of the extracellular matrix in a tumor tissue, including collagen I, collagen III, fibronectin, elastin, or the like and highly expressing a therapeutic gene selectively only in tumor cells through viral proliferation. Particularly, when administered in combination with preexisting anticancer agents or immune checkpoint inhibitors, the recombinant adenovirus significantly increases the diffusion and distribution of the co-administered therapeutic materials in tumor tissues while allowing the exertion of the preexisting anticancer effects, thereby further improving an anti-tumor effect. Accordingly, the present invention can be utilized as a core technique in the cancer treatment field.

### [Description of Drawings]

FIG. 1 is a schematic view schematically illustrating a tumor cell-specific action mechanism of the tumor-selective killing adenovirus according to the present invention.
FIG. 2 is a schematic view schematically illustrating an extracellular matrix (ECM) degradation mechanism of the tumor-selective killing adenovirus according to the present invention.

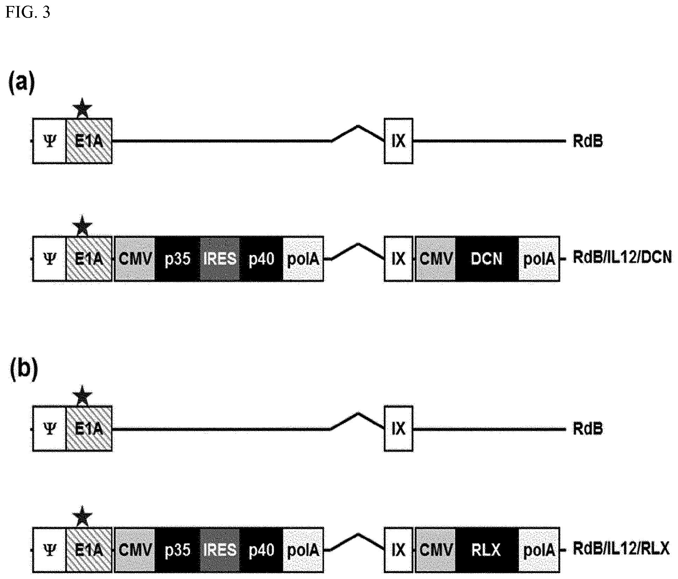

FIG. 3 schematically illustrates the genetic structure of the tumor-selective killing adenovirus according to the present invention, FIG. 3A illustrates the genetic structure of a tumor-selective killing adenovirus (RdB/IL-12/DCN) which simultaneously expresses IL-12 and decorin (DCN), and FIG. 3B illustrates the genetic structure of a tumor-selective killing adenovirus (RdB/IL-12/RLX) which simultaneously expresses IL-12 and relaxin (RLX). Specifically, the RdB includes a modified E1A (open star - mutation of an Rb protein binding site), and E1B-19k and E1B-55k (E1B), and E3 region (E3) are deleted; and IL-12 and DCN/RLX are inserted into E1 and/or E3 site(s) of the adenoviral genome, respectively.
FIG. 4 is a result of confirming, by MTT assay, the ability to kill a pancreatic cancer cell line (PANC-1, MIA PaCa-2, or AsPC-1) by coadministration of RdB/IL-12/DCN and gemcitabine.
FIG. 5 is a result of confirming an anti-tumor effect by coadministration of RdB/IL-12/DCN and gemcitabine through the change in size of tumors formed in an orthotopic pancreatic cancer animal model.
FIG. 6 is a histological evaluation of anti-tumor effects by coadministration of RdB/IL-12/DCN and gemcitabine, and is a result of performing hematoxylin-eosin (H & E) staining, PCNA immunohistological staining, immunohistological staining using an E1A protein antibody, and a TUNEL assay, respectively.
FIG. 7 is a result of confirming an anti-tumor effect by coadministration of RdB/IL-12/DCN and an immune checkpoint inhibitor (anti-PD-L1) through the change in size of tumors formed in a melanoma subcutaneous animal model.
FIG. 8 is a result of confirming an anti-tumor effect by coadministration of RdB/IL-12/DCN and an immune checkpoint inhibitor (anti-PD-L1) through the change in survival rate of a melanoma subcutaneous animal model.
FIG. 9 is a result of confirming an anti-tumor effect by coadministration of RdB/IL-12/DCN and an immune checkpoint inhibitor (anti-PD-L1, anti-PD-L1, or anti-CTLA-4) through the change in size of tumors formed in a hamster subcutaneous pancreatic cancer animal model.
FIG. 10 is a result of confirming a memory immune response (effect of treating a recurrent cancer) by coadministration of RdB/IL-12/DCN and an immune checkpoint inhibitor (anti-PD-L1, anti-PD-L1, or anti-CTLA-4) through the change in size of tumors formed in a hamster subcutaneous pancreatic cancer animal model.
FIG. 11 is a result of confirming an anti-tumor effect by coadministration of RdB/IL-12/DCN and an immune checkpoint inhibitor (anti-PD-L1) through the change in size of tumors formed in an animal model with induced resistance to an anticancer agent.
FIG. 12 is a result of confirming, by MTT assay, the ability to kill a pancreatic cancer cell line (MIA PaCa-2, or PANC-1) by coadministration of RdB/IL-12/RLX and gemcitabine.
FIG. 13 is a result of confirming, by TUNEL assay, an anti-tumor effect by coadministration of RdB/IL-12/RLX and gemcitabine.
FIG. 14 is a result of confirming an extracellular matrix degradation effect by coadministration of RdB/IL-12/RLX and gemcitabine through the change in expression level of collagen I or collagen III.
FIGS. 15A and 15B are results of confirming an extracellular matrix degradation effect by coadministration of RdB/IL-12/RLX and gemcitabine through the change in expression level of collagen I, collagen III, fibronectin, or elastin.
FIGS. 16A and 16B are histological evaluations of an anti-tumor effect by coadministration of RdB/IL-12/RLX and gemcitabine, and are results of performing MT staining, Picrosirius staining, and TUNEL assay, respectively.
FIGS. 17A, 17B and 17C are results of confirming an anti-tumor effect by coadministration of RdB/IL-12/RLX and gemcitabine through the change in size of tumors formed in a pancreatic cancer subcutaneous animal model.
FIG. 18 is a result of confirming an extracellular matrix degradation effect by coadministration of RdB/IL-12/RLX and gemcitabine through the change in expression level of collagen I, collagen IV, fibronectin, or elastin in tumor tissues formed in a pancreatic cancer xenograft animal model.
FIG. 19 is a histological evaluation of an anti-tumor effect by coadministration of RdB/IL-12/RLX and gemcitabine, and is a result of performing hematoxylin-eosin (H & E) staining, TUNEL assay, and cleaved caspase 3 immunostaining, respectively.
FIG. 20 is a result of confirming an anti-tumor effect by coadministration of RdB/IL-12/RLX and an immune checkpoint inhibitor (anti-PD-Ll) through the change in size of tumors formed in a melanoma subcutaneous animal model.
FIG. 21 is a result of confirming an anti-tumor effect by coadministration of RdB/IL-12/RLX and an immune checkpoint inhibitor (anti-PD-L1) through the change in survival rate of a melanoma subcutaneous animal model.
FIG. 22 is a result of confirming an anti-tumor effect by coadministration of RdB/IL-12/RLX and an immune checkpoint inhibitor (anti-PD-L1) through the change in size of tumors formed in a melanoma subcutaneous animal model.
FIG. 23 is a result of confirming an anti-tumor effect by coadministration of RdB/IL-12/RLX and an immune checkpoint inhibitor (anti-PD-L1) through the change in survival rate of a melanoma subcutaneous animal model.
FIG. 24 is a result of confirming an anti-tumor effect by coadministration of RdB/IL-12/RLX (1 × 10⁹ VP) and an immune checkpoint inhibitor (anti-PD-L1) through the change in size of tumors formed in a melanoma subcutaneous animal model.
FIG. 25 is a result of confirming a memory immune response (effect of treating a recurrent cancer) by coadministration of RdB/IL-12/RLX and an immune checkpoint inhibitor (anti-PD-L1, or anti-PD-L1) through the change in size of tumors formed in a melanoma subcutaneous animal model.
FIG. 26 is a result of confirming an anti-tumor effect by coadministration of RdB/IL-12/RLX and an immune checkpoint inhibitor (anti-PD-L1) through the change in size of tumors formed in an animal model with induced resistance to an anticancer agent.
FIG. 27 is a result of confirming an effect of enhancing anticancer agent penetration in a gastric tumor tissue by coadministration of RdB/IL-12/RLX through trastuzumab as an anticancer agent and immunofluorescence staining.
FIGS. 28A, 28B and 28C are evaluations of the distribution of the anticancer agent in a gastric tumor tissue by coadministration of RdB/IL-12/RLX, and are confirmations of the distribution of the anticancer agent based on the margin site in the tumor tissue by enlarging the region indicated in FIG. 27.
FIGS. 29A, 29B and 29C are evaluations of the distribution of the anticancer agent in a gastric tumor tissue by coadministration of RdB/IL-12/RLX, and are confirmations of the distribution of the anticancer agent based on the blood vessels in the tumor tissue.
FIGS. 30A and 30B are evaluation of residence time of the anticancer agent in the gastric tumor tissue by coadministration of RdB/IL-12/RLX, and are results of performing PET imaging.

### [Modes of the Invention]

Hereinafter, the present invention will be described in detail.

The present invention provides a pharmaceutical composition for aiding an anticancer agent, the pharmaceutical composition comprising a recombinant adenovirus comprising a gene encoding Interleukin 12 (IL-12) and a gene encoding decorin or relaxin; a use of the composition for enhancing the therapeutic efficacy of an anticancer agent; and a method for treating cancer, the method comprising a step of administering a therapeutically effective amount of the composition in combination with an anticancer agent.

As used herein, the term "treatment" refers to all actions that ameliorate or beneficially change symptoms (tumors) by administering the pharmaceutical composition according to the present invention.

In the present invention, "an individual" refers to a subject in need of treatment of a disease, and more specifically, refers to a mammal such as a human or a non-human primate, a rodent (a rat, a mouse, a guinea pig, and the like), a dog, a cat, a horse, a cow, a sheep, a pig, a goat, a camel, and an antelope.

"Cancer", which is a disease to be treated by the pharmaceutical composition of the present invention, collectively refers to diseases caused by cells having aggressive characteristics in which the cells ignore normal growth limits and divide and grow, invasive characteristics to infiltrate surrounding tissues, and metastatic characteristics of spreading to other sites in the body. In the present invention, the cancer is used in the same sense as a malignant tumor or tumor, and may include a solid tumor and a blood borne tumor. For example, when classified according to the site of the lesion, the cancer may be any one selected from the group consisting of gastric cancer, lung cancer, non-small cell lung cancer, breast cancer, ovarian cancer, liver cancer, bronchial cancer, nasopharyngeal cancer, laryngeal cancer, pancreatic cancer, bladder cancer, colon cancer, cervical cancer, bone cancer, non-small cell bone cancer, hematologic malignancy, skin cancer, head or neck cancer, uterine cancer, colorectal cancer, anal near cancer, colon cancer, fallopian tube cancer, endometrial cancer, vaginal cancer, vulva cancer, Hodgkin's disease, esophageal cancer, small intestine cancer, endocrine cancer, thyroid cancer, parathyroid cancer, adrenal cancer, soft tissue sarcoma, urethral cancer, penile cancer, prostate cancer, chronic or acute leukemia, lymphocytic lymphoma, kidney or hydroureter cancer, renal cell carcinoma, renal pelvic carcinoma, salivary gland cancer, sarcoma cancer, pseudomyxoma peritonei, hepatoblastoma, testicular cancer, glioblastoma, cheilocarcinoma, ovarian germ cell tumors, basal cell carcinoma, multiple myeloma, gallbladder cancer, choroidal melanoma, cancer of the ampulla of Vater, peritoneal cancer, tongue cancer, small cell cancer, pediatric lymphoma, neuroblastoma, duodenal cancer, ureteral cancer, astrocytoma, meningioma, renal pelvis cancer, pudendum cancer, thymus cancer, central nervous system tumors, primary central nervous system lymphoma, spinal cord tumors, brain stem neuroglioma, and pituitary adenoma, and when classified according to pathological characteristics, the cancer may be a recurrent cancer or an anticancer agent-resistant cancer, but is not limited thereto.

As used herein, the term pharmaceutical composition for aiding an anticancer agent refers to the improvement in cancer treatment efficacy by being used in combination with preexisting cancer therapeutic materials. As an example, the pharmaceutical composition for aiding an anticancer agent indicates the minimization of side effects caused by an anticancer agent by reducing a therapeutically effective dose of the anticancer agent, or the significant inhibition of the growth of tumors. The pharmaceutical composition for aiding an anticancer agent can be used interchangeably with an anticancer adjuvant, an anticancer therapeutic adjuvant, or the like.

In a specific example of the present invention, the anticancer agent may be an antimetabolite, an alkylating agent, a topoisomerase antagonist, a microtubule antagonist, an anticancer antibiotic, a plant-derived alkaloid, an antibody anticancer agent or a molecular targeted cancer agent, and more specifically, may be taxol, nitrogen mustard, imatinib, oxaliplatin, rituximab, erlotinib, trastuzumab, gefitinib, bortezomib, sunitinib, carboplatin, sorafenib, bevacizumab, cisplatin, cetuximab, Viscum album, asparaginase, tretinoin, hydroxycarbamide, dasatinib, estramustine, gemtuzumab ozogamicin, ibritumomab tiuxetan, heptaplatin, methyl aminolevulinic acid, amsacrine, alemtuzumab, procarbazine, alprostadil, holmium nitrate chitosan, gemcitabine, doxifluridine, pemetrexed, tegafur, capecitabine, gimeracil, oteracil, azacitidine, methotrexate, uracil, cytarabine, fluorouracil, fludarabine, enocitabine, decitabine, mercaptopurine, thioguanine, cladribine, carmofur, raltitrexed, docetaxel, paclitaxel, irinotecan, belotecan, topotecan, vinorelbine, etoposide, vincristine, vinblastine, teniposide, doxorubicin, idarubicin, epirubicin, mitoxantrone, mitomycin, bleomycin, daunorubicin, dactinomycin, pirarubicin, aclarubicin, peplomycin, temozolomide, busulfan, ifosfamide, cyclophosphamide, melphalan, altretamine, dacarbazine, thiotepa, nimustine, chlorambucil, mitolactol, lomustine and carmustine, imatinib, gefitinib, erlotinib, tristuzumab, rociletinib, necitumumab, everolimus, ramucirumab, dacomitinib, foretinib, pembrolizumab, ipilimumab, nivolumab, dabrafenib, veliparib, ceritinib, carmustine, cyclophosphamide, ifosfamide, ixabepilone, melphalan, mercaptopurine, mitoxantrone, or TS-1, but is not limited thereto.

The tumor-selective killing recombinant adenovirus included in the pharmaceutical composition of the present invention is characterized by comprising: a gene encoding IL-12; and a gene encoding decorin or relaxin which is a degradation factor for the extracellular matrix.

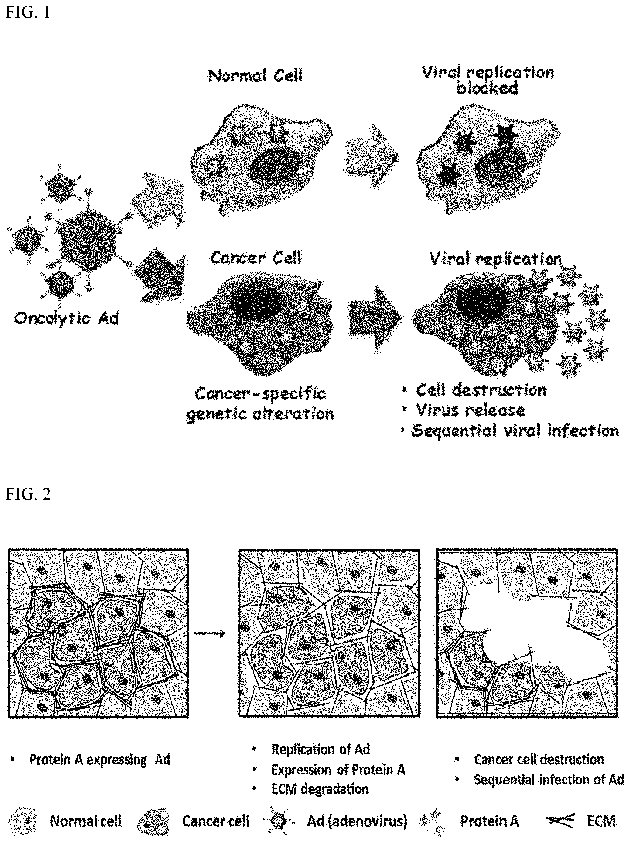

As the recombinant adenovirus of the present invention, an oncolytic adenovirus widely known in the art may be used. As a specific example, the recombinant adenovirus comprises an activated E1A gene and an inactivated E1B-19k gene, an E1B-55k gene, or an E1B-19k/E1B-55k gene. In the present specification, the term inactivation used in conjunction with a gene means that the transcription and/or translation of the gene is not normally performed, and the function of a normal protein encoded by the gene is not exhibited. For example, the inactivated EIB-19k gene is a gene incapable of producing the activated EIB-19k protein by a modification (substitution, addition, and partial or whole deletion) in the gene. The deletion of EIB-19k may increase the ability to kill cells, and the deletion of the E1B-55k gene makes a recombinant adenovirus tumor-specific (see Korean Patent Application No. 2002-23760 and FIG. 1). In particular, the tumor-selective killing adenovirus as described above exhibits therapeutic effects on primary infected cells, the proliferated virus kills tumor cells by secondary and tertiary infection of surrounding tumor cells in a sequential manner, so that the therapeutic effect thereof may continue to spread like a domino phenomenon, and as a result, the anti-tumor effect may be remarkably increased. The term "deletion" used in conjunction with a viral genomic sequence in the present specification has a meaning including complete deletion and partial deletion of the corresponding sequence.

According to an embodiment of the present invention, the recombinant adenovirus includes an E1A region, has an E1B region, that is, E1B-19k and E1B-55k (E1B) deleted, and/or an E3 region (E3) deleted. The recombinant adenovirus including the E1A gene will have replicable characteristics. The gene encoding IL-12 is inserted into the deleted E1 region of the recombinant adenovirus, the gene encoding decorin or relaxin may be inserted into an E1 or E3 region, but the insertion site of the aforementioned gene may be appropriately changed. Meanwhile, the E1A site has a modification in which the Glu 45 residue is substituted with Gly and a modification in which the sequence of amino acids 121 to 127 is wholly substituted with Gly, in a nucleotide sequence encoding an Rb binding site located in the E1A gene sequence.

Meanwhile, viruses other than the adenovirus may also be used in the present invention. The virus which may be used in the present invention may preferably be adeno associated viruses (AAV)(Lashford LS., et al., Gene Therapy Technologies, Applications and Regulations Ed. A. Meager (1999)), retroviruses (Gunzburg WH, et al., Gene Therapy Technologies, Applications and Regulations Ed. A. Meager, (1999)), lentiviruses (Wang G. et al., J. Clin. Invest. 104(11):R55-62(1999)), herpes simplex viruses (Chamber R., et al., Proc. Natl. Acad. Sci USA 92:1411-1415(1995)), vaccinia viruses (Puhlmann M. et al., Human Gene Therapy 10:649-657(1999)), reoviruses, pox viruses (GCE, NJL, Krupa M, Esteban M., The poxvirus vectors MVA and NYVAC as gene delivery systems for vaccination against infectious diseases and cancer Curr Gene Ther 8(2):97-120(2008)), Semliki Forest viruses, and may be applied to polymers (Hwang et al., In vitro and In vivo Transfection Efficiency of Human Osteoprotegerin Gene using Non-Viral Polymer Carriers., Korean J. Bone Metab. 13(2):119-128(2006)), liposomes (Methods in Molecular Biology, Vol 199, S.C. Basu and M. Basu (Eds.), Human Press 2002), nanomaterials or niosomes, but is not limited thereto.

The recombinant adenovirus used in the present invention includes a promoter that is operable in animal cells, preferably mammal cells. A promoter suitable for the present invention includes a promoter derived from a mammalian virus and a promoter derived from the genome of mammalian cells, and includes, for example, a cytomegalovirus (CMV) promoter, a U6 promoter and an H1 promoter, a murine leukemia virus (MLV) long terminal repeat (LTR) promoter, an adenovirus early promoter, an adenovirus late promoter, a vaccina virus 7.5K promoter, an SV40 promoter, a HSV *tk* promoter, an RSV promoter, an EF1 α promoter, a methallothionin promoter, a β-actin promoter, a human IL-2 gene promoter, a human IFN gene promoter, a human IL-4 gene promoter, a human lymphotoxin gene promoter, a human GM-CSF gene promoter, an inducible promoter, a cancer cell-specific promoter (for example, a TERT promoter, a modified TERT promoter, a PSA promoter, a PSMA promoter, a CEA promoter, a Survivin promoter, an E2F promoter, a modified E2F promoter, an AFP promoter, a modified AFP promoter, an E2F-AFP hybrid promoter, and an E2F-TERT hybrid promoter), a tissue-specific promoter (for example, an albumin promoter), a human phosphoglycerate kinase (PGK) promoter, and a mouse phosphoglycerate kinase (PGK) promoter, but is not limited thereto. Most preferably, the promoter is a CMV promoter. It is preferred that in an expression construct for expressing a trans gene, a polyadenylation sequence binds downstream of the trans gene. The polyadenylation sequence includes a bovine growth hormone terminator (Gimmi, E. R., et al., Nucleic Acids Res. 17:6983-6998(1989)), an SV40-derived polyadenylation sequence (Schek, N, et al., Mol. Cell Biol. 12:5386-5393(1992)), HIV-1 polyA (Klasens, B. I. F., et al., Nucleic Acids Res. 26:1870-1876(1998)), β-globin polyA (Gil, A., et al, Cell 49:399-406(1987)), HSV TK polyA (Cole, C. N. and T. P. Stacy, Mol. Cell. Biol. 5:2104-2113(1985)), or polyomavirus polyA (Batt, D. B and G. G. Biol. 15:4783-4790(1995)), but is not limited thereto.

In the recombinant adenovirus used in the present invention, the IL-12 gene sequence and the decorin or relaxin gene sequence are operably linked to a promoter. As used herein, the term "operably linked" refers to a functional binding between a nucleic acid expression regulatory sequence (for example: an array of a promoter, a signal sequence, and a transcription regulating factor-binding site) and a different nucleic acid sequence, and accordingly, the regulatory sequence regulates the transcription and/or translation of the different nucleic acid sequence.

The recombinant adenovirus of the present invention may further include an antibiotic resistance gene and a reporter gene (for example, green fluorescence protein (GFP), luciferase and β-glucuronidase) as selective markers. The antibiotic resistance gene includes an antibiotic resistance gene typically used in the art, for example, a gene imparting resistance to ampicillin, gentamycin, carbenicillin, chloramphenicol, streptomycin, kanamycin, geneticin, neomycin and tetracycline, and preferably, is a neomycin resistance gene. The aforementioned selective marker may be expressed even in an expression system connected by a separate promoter or an internal ribosome entry site (IRES), and the IRES that may be used in the present invention is a regulatory sequence that is found in RNAs of some types of viruses and cells.

The "gene encoding IL-12" used in the present invention includes an IL-12A (p35) gene sequence and an IL-12B (p40) gene sequence, and may include an IRES sequence between the IL-12A (p35) gene sequence and the IL-12B (p40) gene sequence for the effective translation of a viral protein. Preferably, the IL-12A (p35) gene sequence, the IL-12B (p40) gene sequence, and the IRES sequence may consist of SEQ ID No. 1, SEQ ID No. 2, and SEQ ID No. 3, respectively. The IL-12 serves to increase anti-tumor immunity by inducing the differentiation of T helper 1 cells and activating the cytotoxicity of cytotoxic T lymphocytes and natural killer cells.

For the "gene encoding decorin or relaxin" used in the present invention, preferably, the decorin gene sequence and the Relaxin gene sequence may consist of SEQ ID No. 4 and SEQ ID No. 5, respectively. For most tumors showing relatively low therapeutic effects, due to the development of the extracellular matrix (ECM), the adenovirus is not uniformly diffused in a tumor tissue and remains in a localized site of administration in frequent cases. The decorin or relaxin serves to increase the diffusion and distribution of a therapeutic material in a tumor tissue by degrading constituent components of an extracellular matrix, for example, collagen I, collagen III, fibronectin, elastin, or the like (see FIG. 2), and additionally, decorin has its own anti-tumor effect. In particular, the aforementioned effect may also affect the diffusion and distribution of a tumor-selective killing recombinant adenovirus itself and a co-administered material, thereby contributing to the maximization of an anti-tumor effect through the coadministration with a preexisting cancer therapeutic material.

That is, the pharmaceutical composition of the present invention has a technical feature of maximizing the cancer treatment effect by including such an integrated composition to sustainably improve the tumor-selective killing efficacy resulting from the recombinant adenovirus itself and the penetration of an anticancer agent co-administered with the composition in tumor tissues. In other words, it is possible to ultimately obtain therapeutic efficacy close to a perfect cure by remarkably increasing the therapeutic efficacy of an anticancer agent in the related art while maintaining the efficacy of gene therapy itself.

It is interpreted that the gene sequences also include a gene sequence exhibiting substantial identity or substantial similarity. The aforementioned substantial identity refers to a random sequence differing from the sequence of the present invention and having at least 80% homology, more preferably 90% homology, and most preferably 95% homology to the sequence of the present invention, when it is aligned to correspond to the sequence of the present invention as much as possible, and the aligned sequences are analyzed using an algorithm typically used in the art. The aforementioned substantial similarity collectively refers to all of the changes in a gene sequence, such as deletion or insertion of one or more bases, which do not affect the object of the present invention of minimizing homologous recombination with a recombinant adenovirus vector. Therefore, the gene sequence of the present invention is not limited to the sequences of exemplified SEQ ID NOS. 1 to 5, and is interpreted to be included in the scope of the rights of the present invention as long as the sequence does not substantially affect the activity of the final product desired in the present invention.

Further, the pharmaceutical composition of the present invention may be administered simultaneously, separately, or sequentially in combination with an immune checkpoint inhibitor.

As used herein, the term "immune checkpoint" collectively refers to a protein involved in causing stimulating or suppressing signals of an immune response on the surface of immune cells, and cancer cells evade the surveillance network of the immune system by being manipulated such that the stimulation of the immune response and the resulting inhibition of cancer cells are not properly performed through the immune checkpoint, that is, the anti-tumor immune response is neutralized. Preferably, the immune checkpoint protein may be programmed cell death-1 (PD-1), programmed cell death-ligand 1 (PD-L1), programmed cell death-ligand 2 (PD-L2), cluster of differentiation 27 (CD27), cluster of differentiation 28 (CD28), cluster of differentiation 70 (CD70), cluster of differentiation 80 (CD80, also known as B7-1), cluster of differentiation 86 (CD86, also known as B7-2), cluster of differentiation 137 (CD137), cluster of differentiation 276 (CD276), killer-cell immunoglobulin-like receptors (KIRs), lymphocyte-activation gene 3 (LAG3), tumor necrosis factor receptor superfamily, member 4 (TNFRSF4, also known as CD 134), glucocorticoid-induced TNFR-related protein (GITR), glucocorticoid-induced TNFR-related protein ligand (GITRL), 4-1BB ligand (4-1BBL), or cytolytic T lymphocyte associated antigen-4 (CTLA-4), but is not limited thereto.

The immune checkpoint inhibitor is an antagonist or antibody targeting the immune checkpoint protein, and exhibits an anti-tumor effect caused by an immune response by enhancing a protein which stimulates the immune response or blocking a protein which suppresses the immune response. Since the immune checkpoint inhibitor uses an immune response system which is excellent in memory ability in addition to advantages of fewer side effects such as emesis or alopecia than general cytotoxic anticancer agents and large therapeutic effects, the therapeutic effect may be sustained for a long period of time even after the administration of a drug is stopped. However, it has been reported that the immune checkpoint inhibitor has a remarkable reduction in therapeutic effect in the immunosuppression microenvironment, and particularly, tumors recur in some patients.

Meanwhile, the immune gene therapy for tumors has been developed as a promising approach for the past few decades, but tumors also have created numerous other strategies to evade the immune surveillance system. In order to overcome these handicaps and enhance the anti-tumor immunity effect, the present invention constructed a tumor-selective killing adenovirus system including IL-12 and a degradation factor for the extracellular matrix as described above (RdB/IL12/DCN, RdB/IL12/RLX), and provided an environment in which the system according to the present invention may more effectively act by using the tumor-selective killing adenovirus system in combination with an immune checkpoint inhibitor. That is, the efficacy of the immune gene therapy was maximized by blocking the evasion mechanism of tumor cells which neutralize the anti-tumor immune response. According to the remarkable effect, the pharmaceutical composition of the present invention exhibited effective effects for the treatment of not only primary cancer, but also a recurrent cancer or an anticancer agent-resistant cancer which has been pointed out as a limitation of the anticancer agent in the related art.

The pharmaceutical composition of the present invention may comprise a pharmaceutically acceptable carrier in addition to the active ingredient. In this case, the pharmaceutically acceptable carrier is typically used during the formulation, and includes lactose, dextrose, sucrose, sorbitol, mannitol, starch, gum acacia, calcium phosphate, alginate, gelatin, calcium silicate, microcrystalline cellulose, polyvinylpyrrolidinone, cellulose, water, syrup, methylcellulose, methyl hydroxybenzoate, propyl hydroxybenzoate, talc, magnesium stearate, mineral oil, and the like, but is not limited thereto. Furthermore, the pharmaceutically acceptable carrier may further comprise a lubricant, a wetting agent, a sweetening agent, a flavoring agent, an emulsifier, a suspending agent, a preservative, and the like, in addition to the aforementioned ingredients. Suitable pharmaceutically acceptable carriers and formulations are described in detail in Remington's Pharmaceutical Sciences (19th ed., 1995).

The pharmaceutical composition of the present invention may be administered orally or parenterally (for example, intravenously, subcutaneously, intraperitoneally, intraalveolarly, or applied topically), and according to an embodiment of the present invention, the pharmaceutical composition according to the present invention may be preferably directly administered intratumorally. The dose varies depending on the patient's condition and body weight, severity of the disease, drug form, administration route, and duration, but may be suitably selected by those skilled in the art.

The pharmaceutical composition of the present invention is administered in a pharmaceutically effective amount. In the present invention, "pharmaceutically effective amount" means an amount sufficient to treat diseases at a reasonable benefit/risk ratio applicable to medical treatment, and an effective dosage level may be determined according to factors including type of disease of patients, the severity of disease, the activity of drugs, sensitivity to drugs, administration time, administration route, excretion rate, treatment period, and simultaneously used drugs, and other factors well known in the medical field. Another pharmaceutical composition according to the present invention may be administered as an individual therapeutic agent or in combination with other therapeutic agents, may be administered sequentially or simultaneously with therapeutic agents in the related art, and may be administered in a single dose or multiple doses. It is important to administer the composition in a minimum amount that can obtain the maximum effect without any side effects, in consideration of all the aforementioned factors, and this amount may be easily determined by those skilled in the art.

Meanwhile, as another aspect of the present invention, the present invention provides: a pharmaceutical composition for treating cancer, the pharmaceutical composition comprising a recombinant adenovirus comprising a gene encoding Interleukin 12 (IL-12) and a gene encoding decorin or relaxin and administered in combination with an immune checkpoint inhibitor; a use of the composition for treating cancer; and a method for treating cancer, the method comprising a step of co-administering the composition and an immune checkpoint inhibitor to an individual.

Since the pharmaceutical composition for treating cancer according to the present invention, and the like use a technical configuration described in the above-described pharmaceutical composition for aiding an anticancer agent, the description of the content common between the two will be omitted to avoid excessive complexity of the present specification.

Hereinafter, preferred Examples for helping the understanding of the present invention will be suggested. However, the following Examples are provided only to more easily understand the present invention, and the contents of the present invention are not limited by the following Examples.

### [Examples]

### Example 1. Manufacture of tumor-selective killing adenovirus

### 1-1. Manufacture of RdB/IL-12/DCN and RdB/IL-12/RLX

In order to construct an adenoviral shuttle vector which expresses decorin or relaxin in the adenovirus E3 site, pSP72 E3/DCN and pSP72 E3/RLX E3 shuttle vectors were manufactured by cloning a sequence encoding decorin or relaxin in a pSP72 E3/CMV-polA adenovirus E3 shuttle vector (Yun CO. et al., Cancer Gene Ther, 2005. 12(1): p. 61-71). For homologous recombination, pdE1/DCN and pdE1/RLX adenoviral plasmids were manufactured by simultaneously transforming *E. coli* BJ5183 with the pSP72 E3/DCN and pSP72 E3/RLX and each adenoviral total vector pdE1.

In order to construct an Ad E1 shuttle vector which expresses IL-12, a pXC1RdB/IL12 E1 shuttle vector was manufactured by excising a mouse IL-12 gene from pCA14/IL12 (Lee YS. et al., Clin Cancer Res, 2006. 12(19): p. 5859-68) and sub-cloning the gene into a pXC1RdB E1 shuttle vector. For homologous recombination, *E. coli* BJ5183 was transformed simultaneously with the pXC1RdB/IL12 E1 shuttle vector and pdE1/DCN or pdE1/RLX, thereby manufacturing pRdB/IL12/DCN and pRdB/IL12/RLX Ad vectors. The purification, titration, and quality analysis of the adenovirus were carried out according to the prior art (Lee YS. et al., Clin Cancer Res, 2006. 12(19): p. 5859-68, Choi KJ. et al., Gene Ther, 2012. 19(7): p. 711-23).

### Example 2. Use of tumor-selective killing adenovirus expressing decorin in combination with anticancer agent

### 2-1. Confirmation of ability to kill pancreatic cancer cells

When a tumor-selective killing adenovirus expressing relaxin (hereinafter, referred to as RdB/IL-12/DCN) and gemcitabine as a standard therapeutic agent for pancreatic cancer were co-administered, it was intended to verify whether the ability to kill pancreatic cancer cells could be remarkably increased. For this purpose, human pancreatic cancer cell lines PANC-1, MIA PaCa-2, and AsPC-1 were infected with RdB/IL-12/DCN of the present invention at a MOI of 0.5 or 2, and treated together with gemcitabine (0.05, 0.2, 1, 2, 5, 20, or 100 µg/ml), and then the degree of apoptosis was observed through MTT assay.

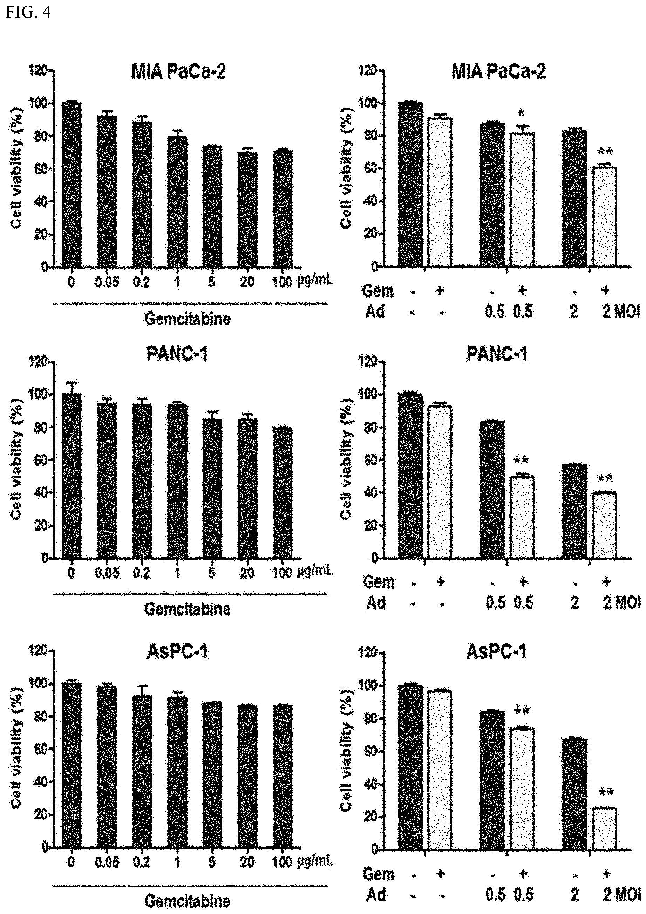

As illustrated in FIG. 4, when treated with gemcitabine alone, all of PANC-1, MIA PaCa-2, and AsPC-1 exhibited the ability to kill at most 30% of the pancreatic cancer cell lines even at a high concentration of 100 µg/ml, but when RdB/IL-12/DCN was administered in combination with gemcitabine, the ability to kill the pancreatic cancer cell lines was remarkably increased even at a low gemcitabine concentration of 0.2 µg/ml. In particular, this effect was excellent as compared to the case where RdB/IL-12/DCN was administered alone, and the higher (2 MOI) the concentration of the recombinant adenovirus was, the more remarkable the effect was. Therefore, the experimental result suggests that co-administration of the preexisting anticancer agent and the RdB/IL-12/DCN according to the present invention may not only reduce the administration dose of the anticancer agent for anti-tumor therapy, but may also exhibit a stronger anti-tumor effect.

### 2-2. Confirmation of anti-tumor effect in orthotopic pancreatic cancer animal model

### (1) Confirmation of change in size of tumor

In order to construct an orthotopic pancreatic cancer animal model, a human pancreatic cancer cell line MIA PaCa-2-Fluc was selected and used in the experiment. Tumor cells were injected into the mouse pancreas, and two weeks later, the production of tumors was confirmed through an IVIS imaging machine. Thereafter, RdB/IL-12/DCN was intraperitoneally administered three times at 2 × 10¹⁰ VP/500 µL every two days, and gemcitabine was administered twice weekly at 100 mg/kg for a total of 3 weeks. Meanwhile, a PBS only administration group was used as a negative control. Before RdB/IL-12/DCN was administered, the size and photon values of tumors were confirmed using the IVIS imaging machine, and at week 1, 2, and 3 after the administration, the changes in size of tumors were quantitatively compared.

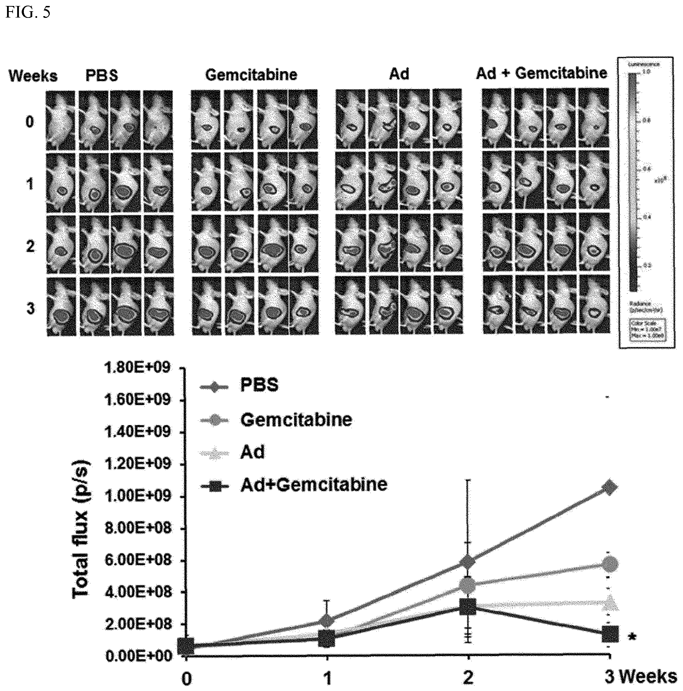

As illustrated in FIG. 5, it could be confirmed that sharp increases in size of tumors were observed in the negative control (PBS) and the gemcitabine only administration group (gemcitabine), whereas in the co-administration group (Ad+ gemcitabine), the growth of tumors was slowed (week 1 and 2), and furthermore, the size of tumors was rather remarkably reduced at week 3. The experimental results indicate that the anti-tumor effect caused by the co-administration of the preexisting anticancer agent and RdB/IL-12/DCN is very remarkable as compared to the respective single administrations.

### (2) Histological evaluation of anti-tumor effect

It was intended to histologically evaluate the anti-tumor effect of the aforementioned experiments. First, the formation of tumors was induced by injecting a human pancreatic cancer cell line MIA PaCa-2-Fluc into the mouse pancreas, and then PBS, gemcitabine, RdB/IL-12/DCN, or gemcitabine+RdB/IL-12/DCN was each administered thereto. Thereafter, hematoxylin-eosin (H & E) staining and PCNA immunohistological staining were performed after collecting the tumor tissues. Further, a TUNEL assay was performed in order to confirm the degree of apoptosis of tumor cells, and immunohistological staining using an antibody against an E1A protein of the adenovirus was performed in order to confirm whether the anti-tumor effect was caused by the replication of the adenovirus present in tumor tissues.

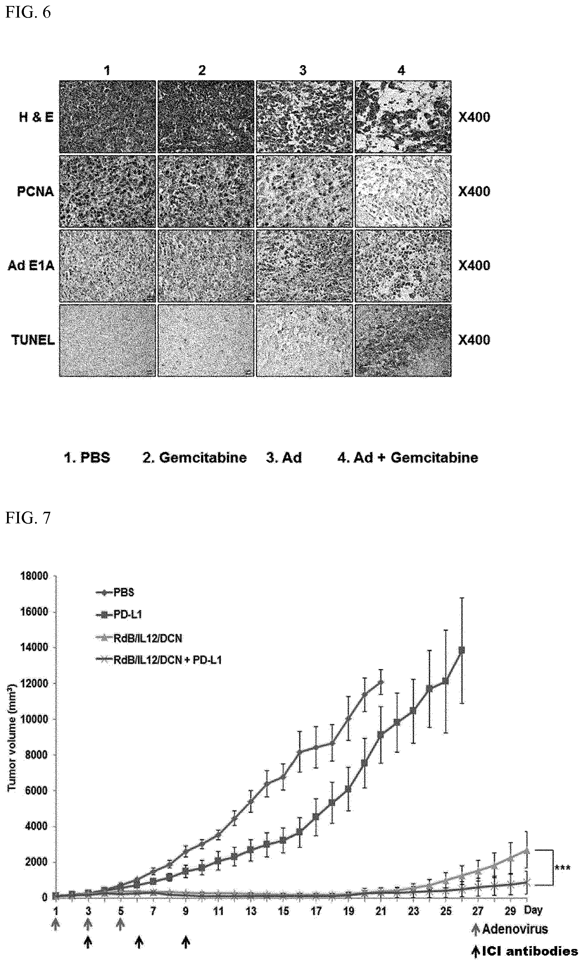

As illustrated in FIG. 6, in the PBS or gemcitabine only administration group, necrosis was hardly observed, whereas when RdB/IL-12/DCN was administered alone or gemcitabine + RdB/IL-12/DCN was co-administered, the necrosis of cells in tumors was very active. In particular, it was confirmed that in the group to which gemcitabine and RdB/IL-12/DCN were co-administered, necrosis occurred in most of the tumors. In addition, as in the above results, it was observed through PCNA immunohistological staining that in the group to which RdB/IL-12/DCN was administered alone or gemcitabine + RdB/IL-12/DCN were administered, the proliferation of tumor cells was remarkably reduced as compared to the group to which PBS or gemcitabine was administered alone, and the effect was the most remarkable in the group to which gemcitabine and RdB/IL-12/DCN were co-administered. Furthermore, with respect to the degree of apoptosis, a high level of apoptosis was also confirmed in the group to which RdB/IL-12/DCN was administered alone or gemcitabine + RdB/IL-12/DCN was administered, particularly, the group to which gemcitabine and RdB/IL-12/DCN were co-administered. Finally, as a result of carrying out immunohistological staining using an antibody against an F1A protein of the adenovirus, it could be confirmed that the E1A protein of the adenovirus was remarkably increased in tumors of the mouse to which RdB/IL-12/DCN was administered alone or gemcitabine + RdB/IL-12/DCN was administered as compared to tumors of the mouse to which only PBS or gemcitabine was administered.

When these results are taken together, the distribution of RdB/IL-12/DCN in tumor tissues is increased by decorin, and accordingly, the active replication of the tumor-selective killing adenovirus and the killing of tumor cells are induced. Further, it can be seen that as the decorin gene is expressed at a high level by the active proliferation of RdB/IL-12/DCN distributed in tumor tissues, the apoptosis of tumor cells was again promoted, and a strong anti-tumor effect was caused by enhancing the efficacy of the co-administered anticancer agent.

### Example 3. Use of tumor-selective killing adenovirus expressing decorin in combination with immune checkpoint inhibitor

### 3-1. Confirmation of anti-tumor effect in melanoma subcutaneous animal model

After a melanoma cell line B16-F10 was injected subcutaneously into mice at 5 x 10⁵ cells/50 µL per animal, about 10 days later, when the size of tumors reached about 100 mm³, the change in size of tumors was observed while 5 x 10⁹ VP of RdB/IL-12/DCN was administered alone or in combination with an immune checkpoint inhibitor (anti-PD-L1) at a potency of 200 µg. During the co-administration, RdB/IL-12/DCN was intratumorally administered on day 1, 3, and 5 (three times in total), and the immune checkpoint inhibitor was intraperitoneally administered on day 3, 6, and 9 (three times in total). Meanwhile, a PBS only administration group was used as a negative control.

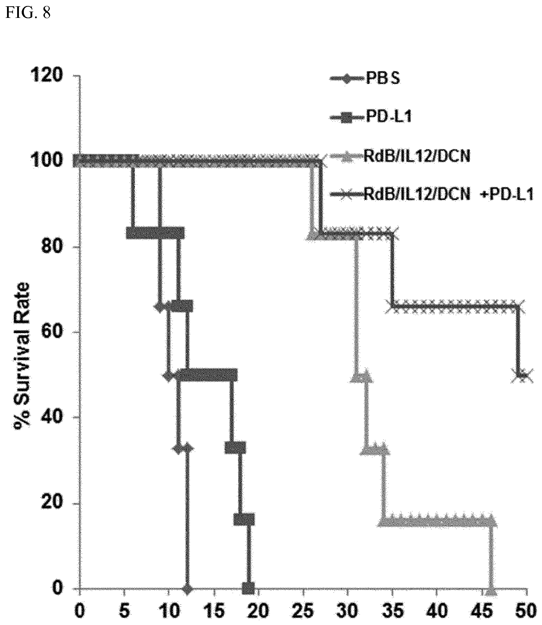

As illustrated in FIGS. 7 and 8, it was confirmed that when the tumor-selective killing adenovirus (RdB/IL-12/DCN) was administered in combination with the immune checkpoint inhibitor, an increase in size of tumors was remarkably reduced, and the survival rate of the melanoma subcutaneous animal model was significantly improved as compared to the RdB/IL-12/DCN or immune checkpoint inhibitor only administration group. Therefore, the experimental results indicate that the co-administration of the RdB/IL-12/DCN according to the present invention and the immune checkpoint inhibitor may be used as a stronger and more effective anticancer therapeutic agent.

### 3-2. Confirmation of anti-tumor effect in hamster subcutaneous pancreatic cancer animal model

It was intended to confirm an anti-tumor immune response by co-administration of RdB/IL-12/DCN and an immune checkpoint inhibitor using a hamster model capable of in vivo replication of the recombinant virus while possessing an in vivo immune function. After a hamster pancreatic cancer cell line Hap-T1 was injected subcutaneously into a hamster, when the size of tumors reached about 100 mm³, the change in size of tumors was observed while 2 x 10⁹ VP of RdB/IL-12/DCN was administered alone or in combination with an immune checkpoint inhibitor (anti-PD-L1, anti-PD-L1, or anti-CTLA-4) at a potency of 700 µg. During the co-administration, RdB/IL-12/DCN was intratumorally administered three times in total at an interval of 2 days, and the immune checkpoint inhibitor was intraperitoneally administered three times in total. Meanwhile, a PBS only administration group was used as a negative control.

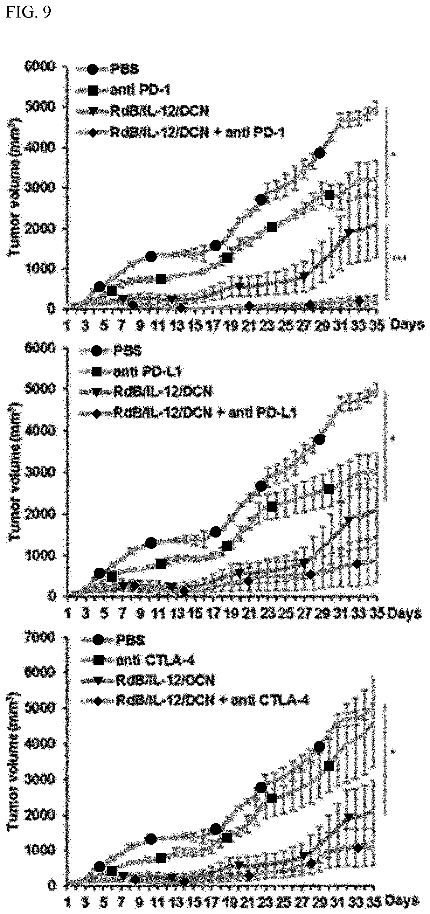

As illustrated in FIG. 9, as a result of administering a tumor-selective killing adenovirus (RdB/IL-12/DCN) in combination with an immune checkpoint inhibitor, an increase in size of tumors was remarkably reduced as compared to the RdB/IL-12/DCN or immune checkpoint inhibitor only administration group, and this trend was equally exhibited for all of the three immune checkpoint inhibitors (anti-PD-1, anti-PD-L1, or anti-CTLA-4). Specifically, after 21 days of administration, the immune checkpoint inhibitors (anti-PD-1, anti-PD-L1, or anti-CTLA-4) inhibited the growth of tumors by only about 35.4%, 38.8%, or 7.2%, respectively, as compared to the negative control, but the RdB/IL-12/DCN only administration group could inhibit the growth of tumors by as much as about 89.8%, 57.7%, or 48.2%, respectively. In particular, the co-administration of RdB/IL-12/DCN and the immune checkpoint inhibitor could inhibit the growth of tumors by as much as 95.7%, 82.1%, or 78.1%, respectively as compared to the negative control, so that the above-described anti-tumor effect could be verified again.

### 3-3. Confirmation of memory immune response in hamster subcutaneous pancreatic cancer animal model

It was intended to verify an anti-tumor memory immune response by the co-administration of the tumor-selective killing adenovirus and the immune checkpoint inhibitor. 49 days after the first tumor was produced, a tumor was formed again in mice from which the tumor had completely disappeared (the RdB/IL-12/DCN only administration group, or the co-administration group of RdB/IL-12/DCN and the immune checkpoint inhibitor) by injecting Hap-T1 cells into the mice under the same conditions as the previous conditions. Thereafter, the growth of tumors was observed in the same manner as in the previous case while administering RdB/IL-12/DCN alone or RdB/IL-12/DCN in combination with the immune checkpoint inhibitor (anti-PD-1, anti-PD-L1, or anti-CTLA-4).

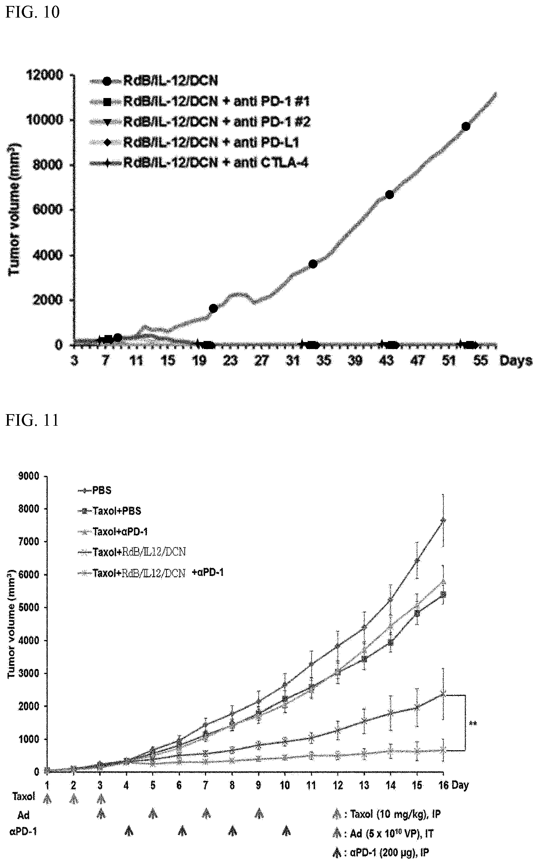

As illustrated in FIG. 10, it was confirmed that in the group treated with RdB/IL-12/DCN alone, a rechallenged tumor was rapidly grown again, whereas when RdB/IL-12/DCN and the immune checkpoint inhibitor (anti-PD-1, anti-PD-L1, or anti-CTLA-4) were co-administered, the growth of tumors was still inhibited. Therefore, the experimental results indicate that the co-administration of the RdB/IL-12/DCN according to the present invention and the immune checkpoint inhibitor may also be effective for the treatment of a recurrent cancer by inducing a strong anti-tumor memory immune response.

### 3-4. Confirmation of anti-tumor effect in animal model with induced resistance to anticancer agent

Under the conditions where the anti-tumor effect caused by an anticancer agent is insignificant due to the resistance to the anticancer agent, and the like, it was intended to verify an in vivo anti-tumor effect by the co-administration of RdB/IL-12/DCN and the immune checkpoint inhibitor (an anti PD-1 antibody; αPD-1). After a B16-F10 mouse melanoma cell line was injected (5 x 10⁵ cells) subcutaneously into the flank of C57BL/6 mice, taxol (day 1, 2, and 3; 10 mg/kg) was injected intraperitoneally into the formed tumor (day 1, about 50 mm³), and after RdB/IL-12/DCN was administered alone (day 3, 5, 7, and 9; 5 × 10⁵ VP), αPD-1 was administered alone (day 4, 7, 8, and 10; 200 µg), or RdB/IL-12/DCN and αPD-1 were co-administered (under the same conditions, respectively), the growth of tumors was observed. Meanwhile, a PBS only administration group was used as a negative control.

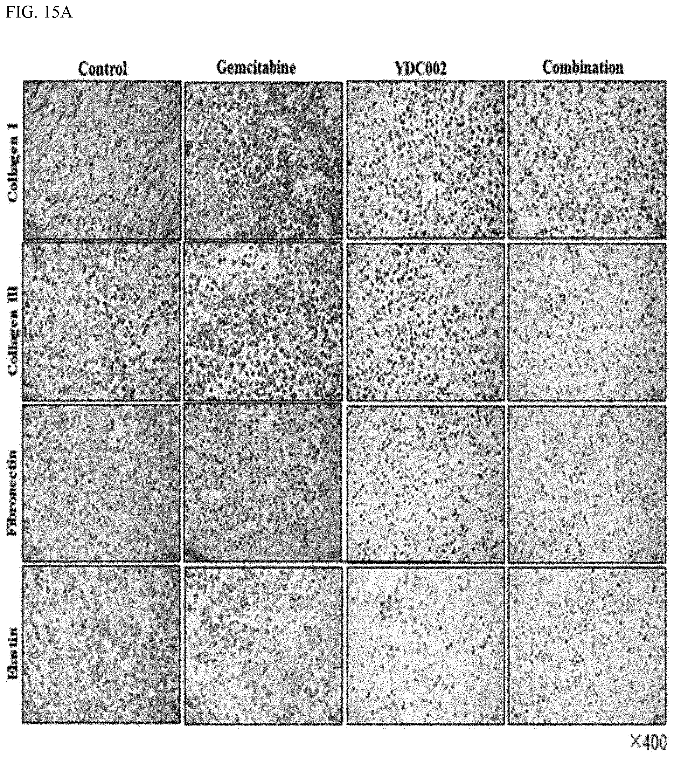

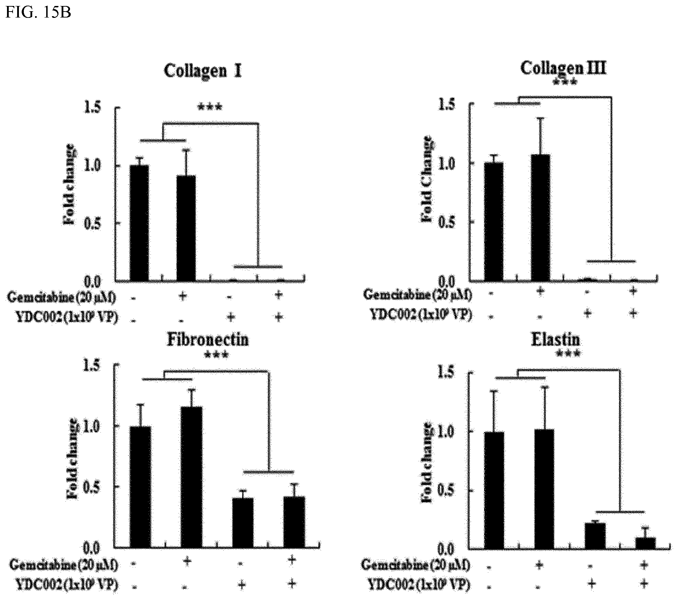

As illustrated in FIG. 11, 15 days after the first administration of the anticancer agent (i.e., on day 16), when PBS, taxol, or αPD-1 was administered alone, the size of tumors was 7649.0 ± 798.5 mm³, 5394.5 ± 288.2 mm³, or 5814.2 ± 471.6 mm³, respectively, confirming the rapid growth of tumors, whereas in the RdB/IL-12/DCN only administration group, or the co-administration group of RdB/IL-12/DCN and αPD-1, the size of tumors was 2374.0 ± 776.2 mm³ or 669.8 ± 335.2 mm³, respectively, exhibiting a significant anti-tumor effect. In particular, in the co-administration group of RdB/IL-12/DCN and αPD-1, the size of tumors was reduced by about 71.8% as compared to the RdB/IL-12/DCN only administration group, so that the effect was very significant (*P* < 0.01). These experimental results exhibit a remarkable effect that the co-administration of the RdB/IL-12/DCN according to the present invention and the immune checkpoint inhibitor not only more improves the anti-tumor effect than that of the preexisting anticancer agents, but also imparts a significant anti-tumor effect even when the effect of the anticancer agent is insignificant due to in vivo factors such as resistance to an anticancer agent.

### Example 4. Use of tumor-selective killing adenovirus expressing relaxin in combination with anticancer agent

### 4-1. Confirmation of ability to kill pancreatic cancer cells

When a tumor-selective killing adenovirus expressing relaxin and a pancreatic cancer standard therapeutic agent gemcitabine were co-administered, it was intended to verify whether the ability to kill pancreatic cells could be remarkably increased. For this purpose, after pancreatic cancer cell lines MIA PaCa-2 and PANC-1 were infected with RdB/IL-12/RLX at a MOI of 0.1, 0.5, 1, or 2 and simultaneously treated with gemcitabine at 0.01 µM or 0.05 µM, the degree of apoptosis was observed through MTT assay. Meanwhile, a non-treatment group was used as a negative control.

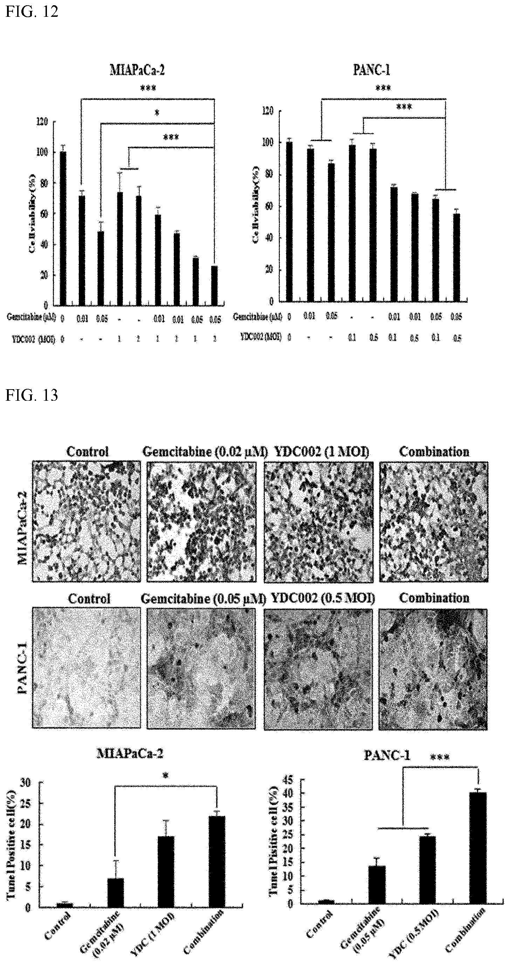

As illustrated in FIG. 12, when RdB/IL-12/RLX (YDC002) was administered in combination with gemcitabine, the ability to kill pancreatic cancer cell lines was remarkably increased as compared to the case of treatment with gemcitabine alone. In particular, this effect was excellent as compared to the case where RdB/IL-12/RLX was administered alone, and the higher (2 MOI) the concentration of the recombinant adenovirus was, the more remarkable the effect was. Therefore, the experimental result suggests that co-administration of the preexisting anticancer agent and the RdB/IL-12/RLX according to the present invention may not only reduce the administration dose of the anticancer agent for anti-tumor therapy, but may also exhibit a stronger anti-tumor effect.

### 4-2. Histological evaluation of anti-tumor effect

It was intended to histologically evaluate the anti-tumor effect of the aforementioned experiments. First, after RdB/IL-12/RLX was administered alone (0.5 or 1 MOI), gemcitabine was administered alone (0.02 or 0.05 µM), or RdB/IL-12/RLX and gemcitabine were co-administered to a pancreatic cancer cell line MIA PaCa-2 or PANC-1, a TUNEL assay was performed in order to confirm the degree of apoptosis of tumor cells. Meanwhile, a non-treatment group was used as a negative control. Further, it was intended to confirm a change in extracellular matrix (ECM) by RdB/IL-12/RLX in the pancreatic cancer cell line. In order to confirm the expression levels of mRNAs of collagen I and collagen III in the tumor cells, qPCR was performed, and the change in extracellular matrix in pancreatic cancer tissues (spheroids) and the apoptosis effect caused by the change were confirmed through immunohistological staining, MT staining, Picrosirius staining, and the like.

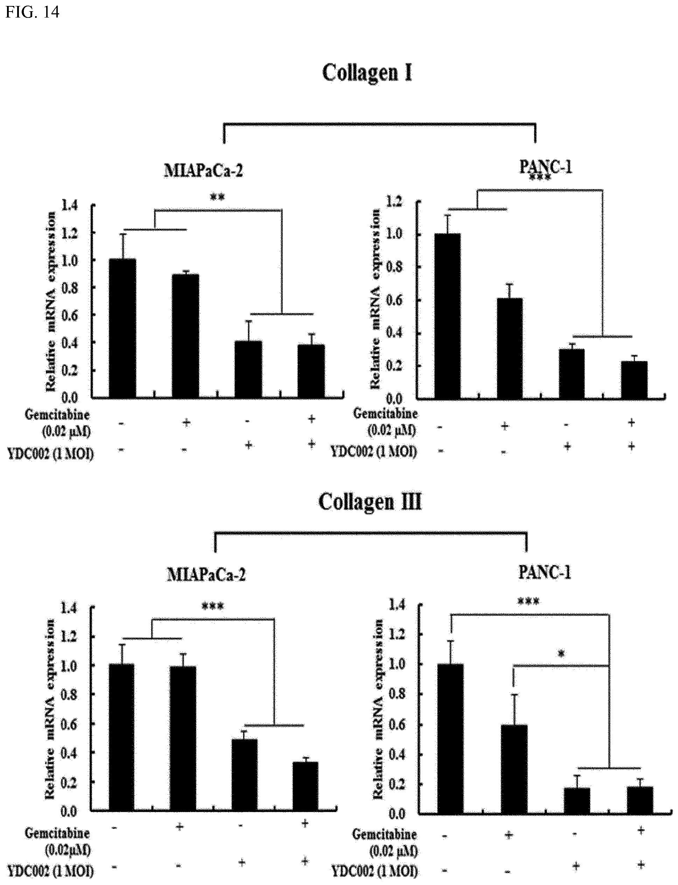

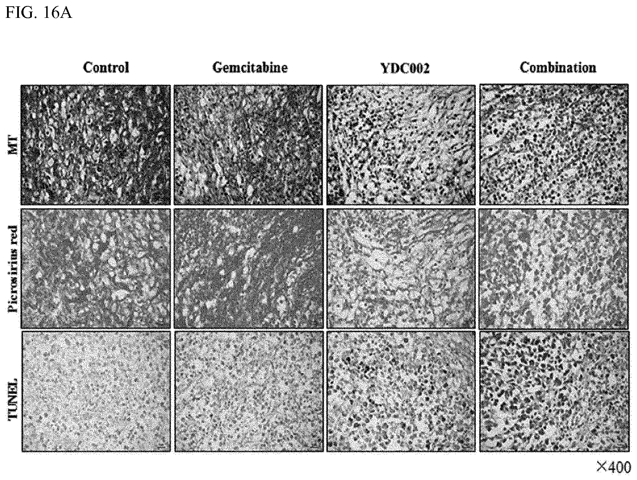

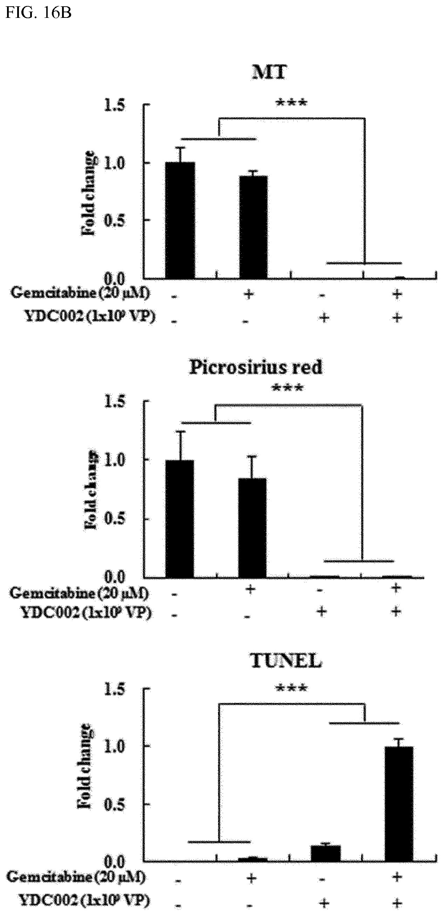

As illustrated in FIG. 13, in both MIA PaCa-2 and PANC-1 cells, it was observed that the apoptosis of tumor cells was remarkably increased in the RdB/IL-12/RLX only administration group or the co-administration group of RdB/IL-12/RLX and gemcitabine as compared to the control or the gemcitabine only administration group, and the effect was the most remarkable in the co-administration group of RdB/IL-12/RLX and gemcitabine. As a result of comparing the expression levels of mRNAs of collagen I and collagen III at 72 hours after human pancreatic cancer cell lines MIA PaCa-2 and PANC-1 were infected with RdB/IL-12/RLX at an MOI of 1, as illustrated in FIG. 14, the expression levels of mRNAs were remarkably decreased in the RdB/IL-12/RLX only administration group or the co-administration group of RdB/IL-12/RLX and gemcitabine as compared to those in the other groups. Likewise, as illustrated in FIGS. 15A, 15B, 16A, and 16B, it was confirmed that in the RdB/IL-12/RLX only administration group, or the co-administration group of RdB/IL-12/RLX and gemcitabine, the expression levels of collagen I, collagen III, fibronectin, and elastin were greatly decreased, and accordingly, it was confirmed that the apoptosis of tumor cells was greatly improved.

When these results are taken together, it can be seen that the expression of extracellular matrix proteins was decreased by relaxin, and accordingly, the anti-tumor effect is maximized by enhancing the efficacy of the cancer-selective killing adenovirus and the co-administered anticancer agent.

### 4-3. Confirmation of anti-tumor effect in pancreatic cancer subcutaneous animal model

It was intended to verify the anti-tumor effect by the co-administration of RdB/IL-12/RLX and gemcitabine in a pancreatic cancer subcutaneous animal model. The formation of tumors was induced by injecting PANC-1, MIA PaCa-2, or BxPC-3 cells subcutaneously into mice, a change in size of tumors was observed while RdB/IL-12/RLX was administered alone or RdB/IL-12/RLX was administered (administered intratumorally) in combination with gemcitabine (10 mg/kg). Meanwhile, a PBS only administration group was used as a negative control.

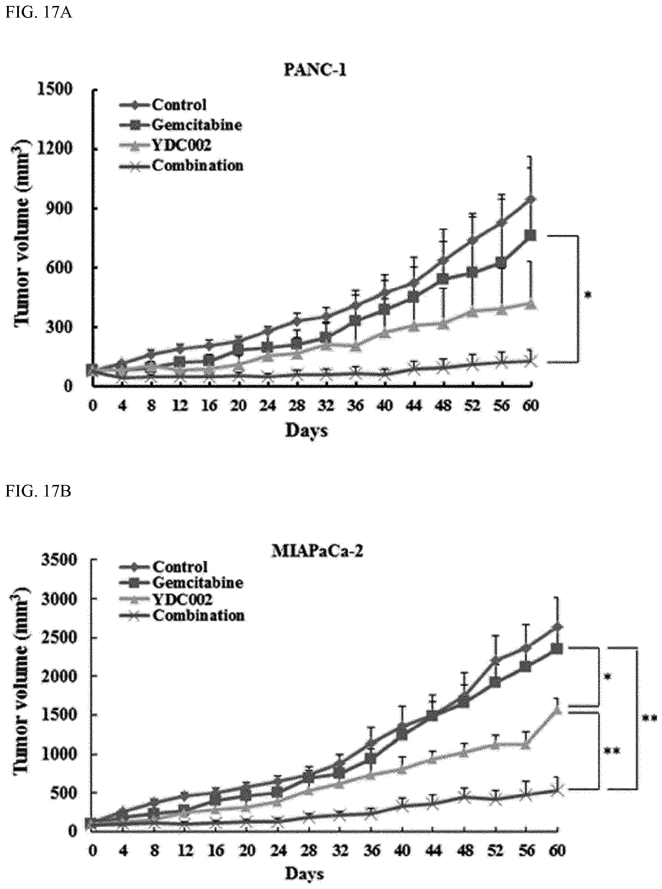

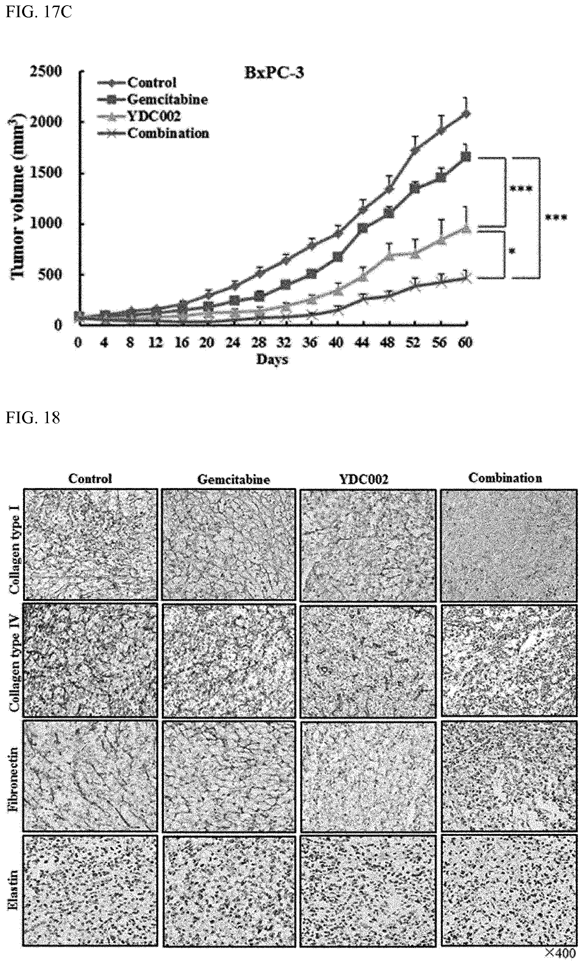

As illustrated in FIGS. 17A to 17C, commonly in all the pancreatic cancer subcutaneous animal models (PANC-1, MIA PaCa-2, or BxPC-3), a sharp increase in size of tumors was observed in the negative control (PBS) and the gemcitabine only administration group (Gemcitabine), whereas it was confirmed that in the RdB/IL-12/RLX only administration group or the co-administration group of RdB/IL-12/RLX and gemcitabine, the growth of tumors was greatly slowed, and particularly, the effect was very remarkable in the case of the co-administration. The experimental results indicate that the anti-tumor effect caused by the co-administration of the preexisting anticancer agent and RdB/IL-12/RLX is very remarkable as compared to the respective single administrations.

### 4-4. Confirmation of anti-tumor effect in pancreatic cancer xenograft animal model

In order to construct a pancreatic cancer xenograft animal model, 5 x 10⁵ of MIA PaCa-2 were injected subcutaneously into mice, and then when the size of tumors reached 50 to 100 mm³, a change in extracellular matrix and an apoptosis effect were observed while 2 x 10⁶ pfu of RdB/IL-12/RLX were administered alone or administered in combination with 1.5 mg/kg of gemcitabine. During the co-administration, RdB/IL-12/RLX was intratumorally administered three times in total at an interval of 2 days, and gemcitabine was intraperitoneally administered once. Thereafter, immunohistological staining and hematoxylin-eosin (H & E) staining were performed, and in order to confirm the degree of apoptosis, a TUNEL assay and cleaved caspase 3 immunostaining were performed. Meanwhile, a PBS only administration group was used as a negative control.

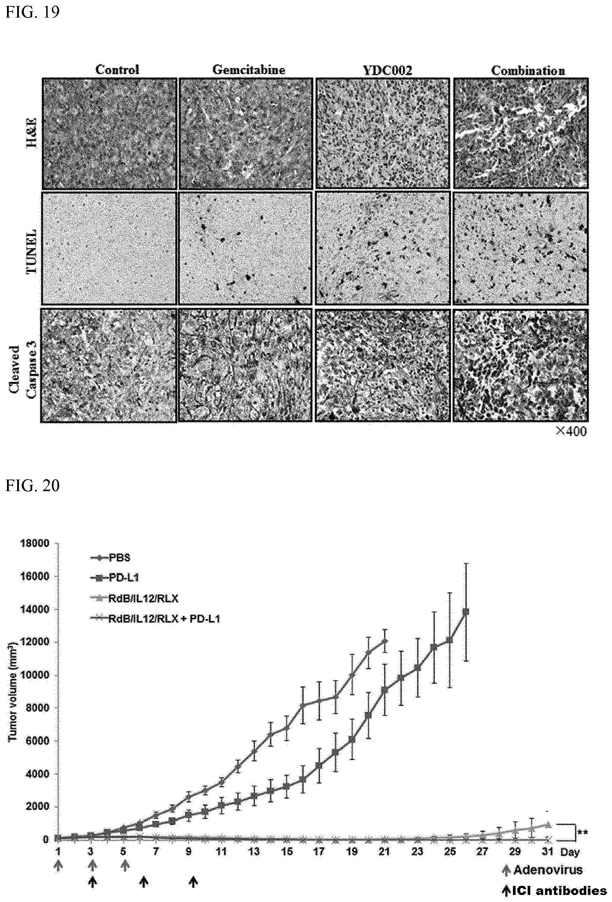

As illustrated in FIG. 18, in the co-administration group of RdB/IL-12/RLX and gemcitabine, all of collagen I, collagen IV, fibronectin, and elastin in MIA PaCa-2 tumor tissues were remarkably reduced as compared to the other groups to which RdB/IL-12/RLX and gemcitabine were co-administered. Further, as illustrated in FIG. 19, it could be seen that in the negative control and the gemcitabine or RdB/IL-12/RLX only administration group, the necrosis or apoptosis of cells in tumors almost hardly proceeded or was insignificant, whereas the necrosis or apoptosis was active in the co-administration group of RdB/IL-12/RLX and gemcitabine. That is, the experimental result again verifies the anti-tumor effect caused by the co-administration of the anticancer agent and the RdB/IL-12/RLX according to the present invention.

### Example 5. Use of tumor-selective killing adenovirus expressing relaxin in combination with immune checkpoint inhibitor

### 5-1. Confirmation of anti-tumor effect in melanoma subcutaneous animal model

After a melanoma cell line B16-F10 was injected subcutaneously into mice at 5 x 10⁵ cells/50 µL per animal, about 7 to 14 days later, when the size of tumors reached about 100 to 150 mm³, the change in size of tumors and the survival rate of the animal model were evaluated while 5 x 10⁹ VP of RdB/IL-12/RLX was administered alone or in combination with an immune checkpoint inhibitor (anti-PD-L1 or anti-PD1) at a potency of 200 µg. During the co-administration, RdB/IL-12/RLX was intratumorally administered three on day 1, 3, and 5 (times in total), and the immune checkpoint inhibitor was intraperitoneally administered on day 3, 6, and 9 (three times in total). Meanwhile, a PBS only administration group was used as a negative control.

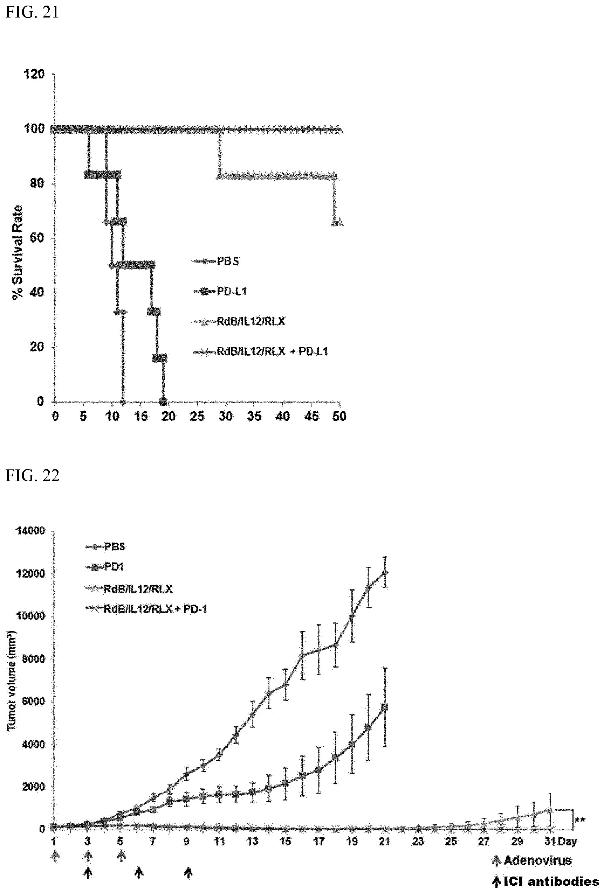

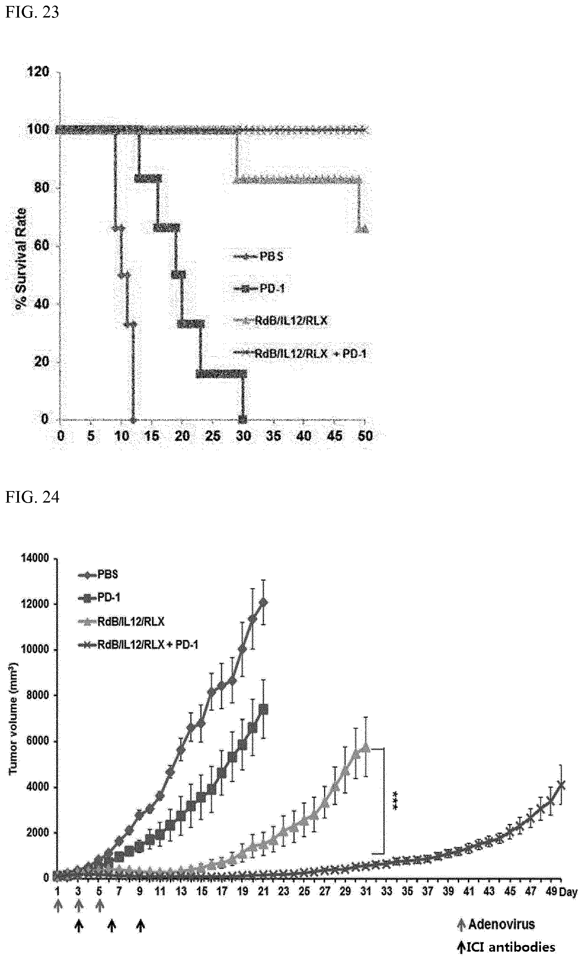

As illustrated in FIGS. 20 to 23, it was confirmed that as a result of co-administering the tumor-selective killing adenovirus (RdB/IL-12/RLX) and the immune checkpoint inhibitor (anti-PD-L1 or anti-PD1), the increase in size of tumors was remarkably decreased, and the survival rate of the melanoma subcutaneous animal model was significantly improved as compared to the RdB/IL-12/RLX or immune checkpoint inhibitor only administration group. Further, in the aforementioned experiment, the anti-tumor effect caused by the single administration of a tumor-selective killing adenovirus RdB/IL-12/RLX was so strong that it was difficult to prove an excellent effect of the co-administration. Accordingly, as a result of performing a re-experiment by decreasing the administration concentration of RdB/IL-12/RLX 5-fold (1 × 10⁹ VP), as illustrated in FIG. 24, it could be confirmed that the anti-tumor effect of the group to which RdB/IL-12/RLX and the immune checkpoint inhibitor (anti-PD1) were co-administered was remarkable as compared to that of the RdB/IL-12/RLX only administration group. Therefore, the experimental results indicate that the co-administration of the RdB/IL-12/IL according to the present invention and the immune checkpoint inhibitor may be used as a stronger and more effective anticancer therapeutic agent.

### 5-2. Confirmation of memory immune response in melanoma subcutaneous animal model

It was intended to verify an anti-tumor memory immune response by the co-administration of the tumor-selective killing adenovirus and the immune checkpoint inhibitor. 50 days after the first tumor was produced, a tumor was formed again in mice from which the tumor had completely disappeared (a co-administration group of RdB/IL-12/RLX and the immune checkpoint inhibitor) by injecting B16-F10 cells into the mice under the same conditions as the previous conditions. Thereafter, the growth of tumors was observed in the same manner as in the previous case while administering RdB/IL-12/RLX alone or RdB/IL-12/RLX in combination with the immune checkpoint inhibitor (anti-PD-1 and anti-PD-L1).

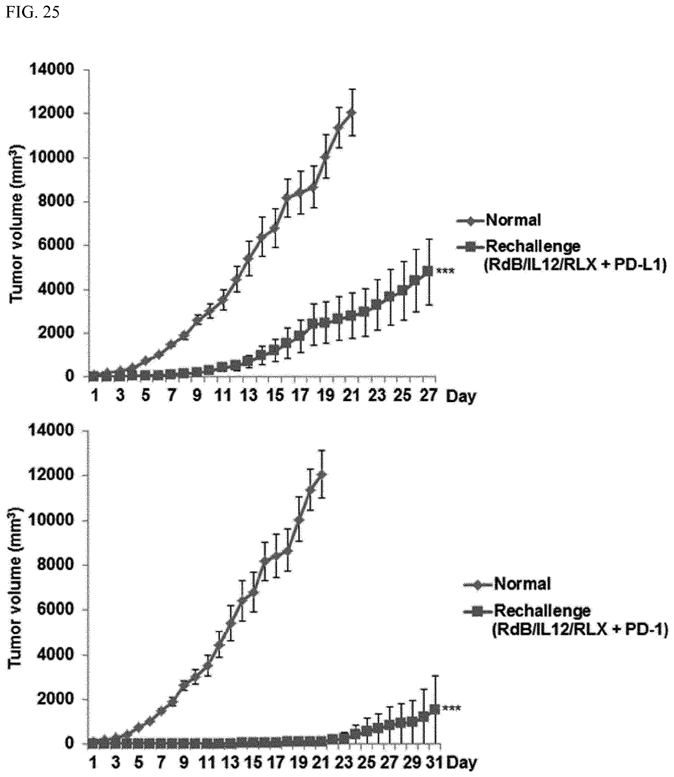

As illustrated in FIG. 25, it was confirmed that when RdB/IL-12/RLX and the immune checkpoint inhibitor (anti-PD-1 and anti-PD-L1) were co-administered, the growth of tumors was sharply reduced. Therefore, the experimental results indicate that the co-administration of the RdB/IL-12/RLX according to the present invention and the immune checkpoint inhibitor may also be effective for the treatment of a recurrent cancer by inducing a strong anti-tumor memory immune response.

### 5-3. Confirmation of anti-tumor effect in animal model with induced resistance to anticancer agent

Under the conditions where the anti-tumor effect caused by an anticancer agent is insignificant due to the resistance to the anticancer agent, and the like, it was intended to verify an in vivo anti-tumor effect by the co-administration of RdB/IL-12/RLX and the immune checkpoint inhibitor (an anti PD-1 antibody; αPD-1). After a B16-F10 mouse melanoma cell line was injected (5 x 10⁵ cells) subcutaneously into the flank of C57BL/6 mice, taxol (day 1, 2, and 3; 10 mg/kg) was injected intraperitoneally into the formed tumor (day 1, about 50 mm³), and after RdB/IL-12/DCN was administered alone (day 3, 5, 7, and 9; 5 × 10⁵ VP), αPD-1 was administered alone (day 4, 7, 8, and 10; 200 µg), or RdB/IL-12/DCN and αPD-1 were co-administered (under the same conditions, respectively), the growth of tumors was observed. Meanwhile, a PBS only administration group was used as a negative control.

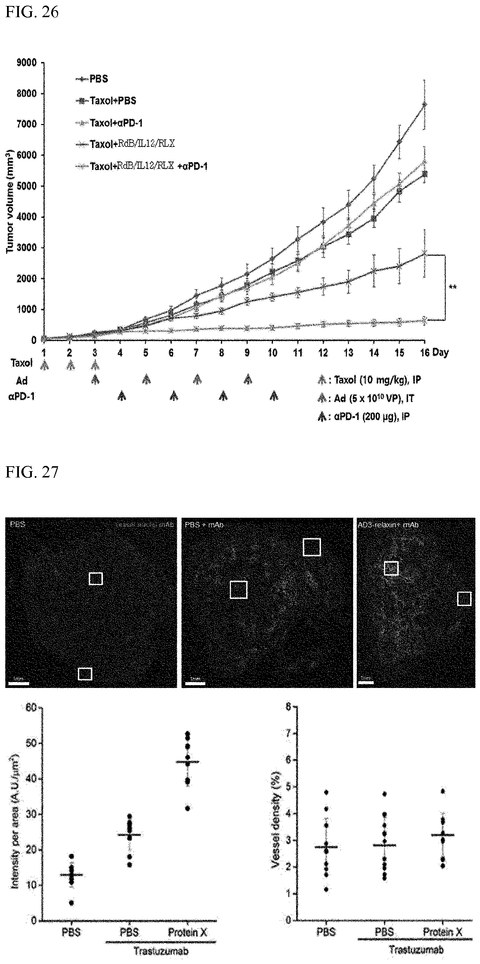

As illustrated in FIG. 26, 15 days after the first administration of the anticancer agent (i.e., on day 16), when PBS, taxol, or αPD-1 was administered alone, the size of tumors was 7649.0 ± 798.5 mm³, 5394.5 ± 288.2 mm³, or 5814.2 ± 471.6 mm³, respectively, confirming the rapid growth of tumors, whereas in the RdB/IL-12/RLX only administration group, or the co-administration group of RdB/IL-12/RLX and αPD-1, the size of tumors was 2817.1 ± 776.2 mm³ or 633.9 ± 141.9 mm³, respectively, exhibiting a significant anti-tumor effect. In particular, in the co-administration group of RdB/IL-12/RLX and αPD-1, the size of tumors was reduced by about 77.5% as compared to the RdB/IL-12/RLX only administration group, so that the effect was very significant (P < 0.01). These experimental results exhibit a remarkable effect that the co-administration of the RdB/IL-12/RLX according to the present invention and the immune checkpoint inhibitor not only more improves the anti-tumor effect than that of the preexisting anticancer agents, but also imparts a significant anti-tumor effect even when the effect of the anticancer agent is insignificant due to in vivo factors such as resistance to an anticancer agent.

### Example 6. Confirmation of effect of tumor-selective killing adenovirus expressing relaxin on tumor tissue infiltration

It was intended to confirm the degree of infiltration and diffusion of the therapeutic material into tumor tissues by performing immunofluorescence staining. After PBS was administered alone, and PBS and trastuzumab (150 µg/mice) or RdB/IL-12/RLX (2.5 × 10¹⁰ VP) and trastuzumab were co-administered to NCIN87 gastric tumor tissues, the infiltration and distribution aspects of trastuzumab labeled with Alexa488 in tumor tissues were compared.

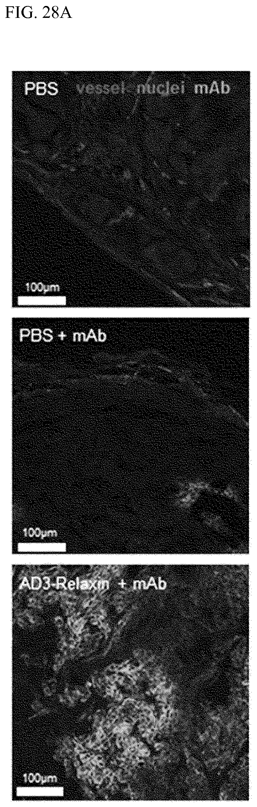

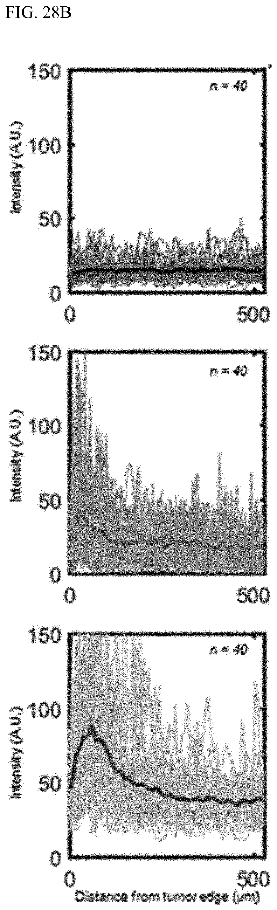

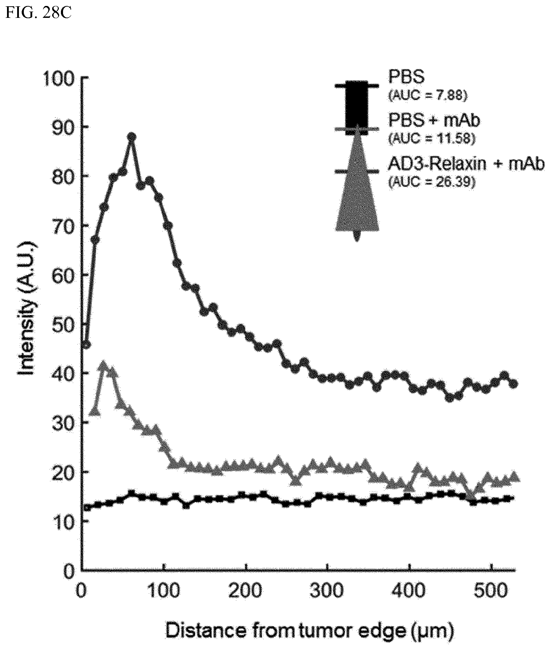

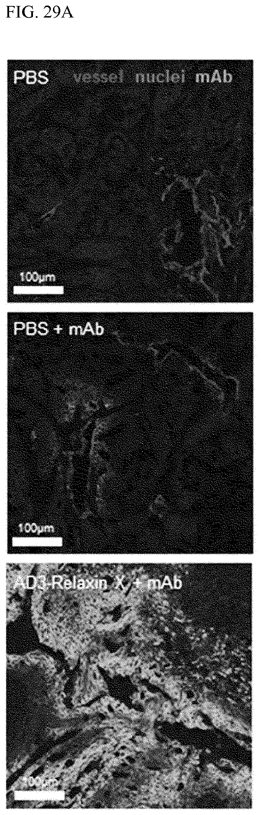

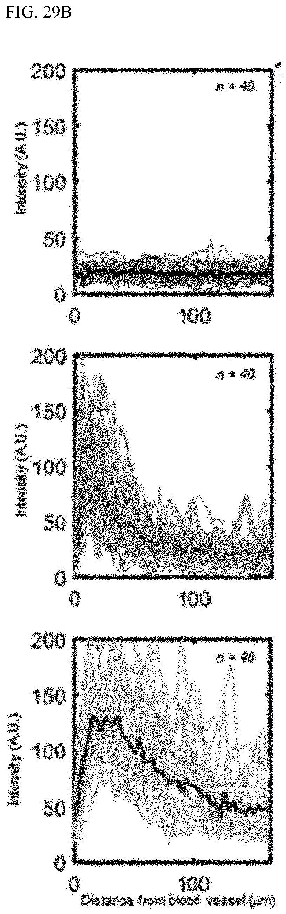

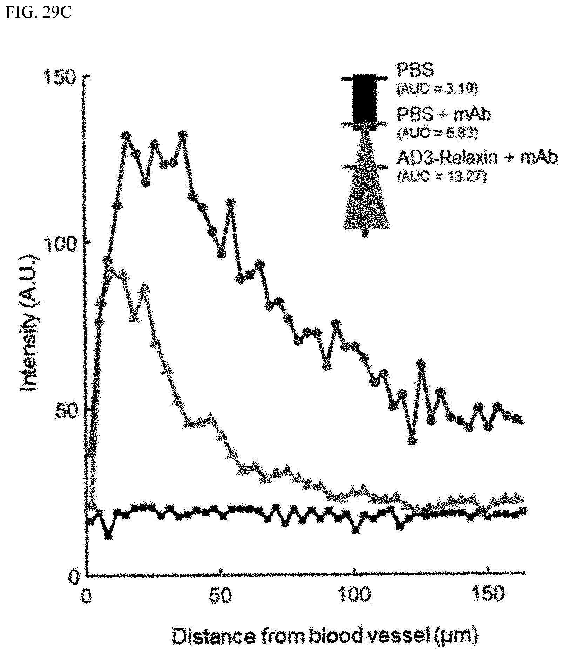

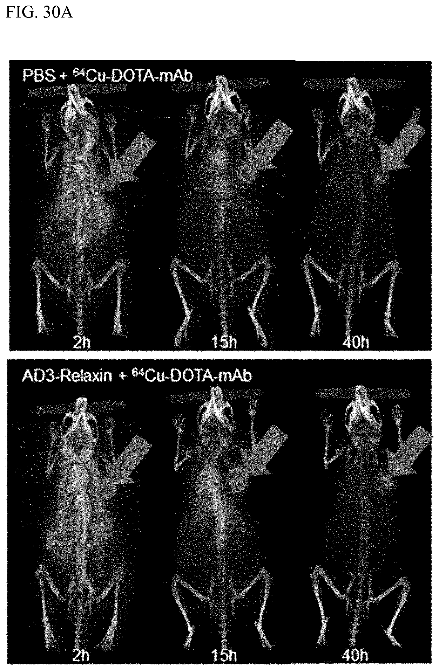

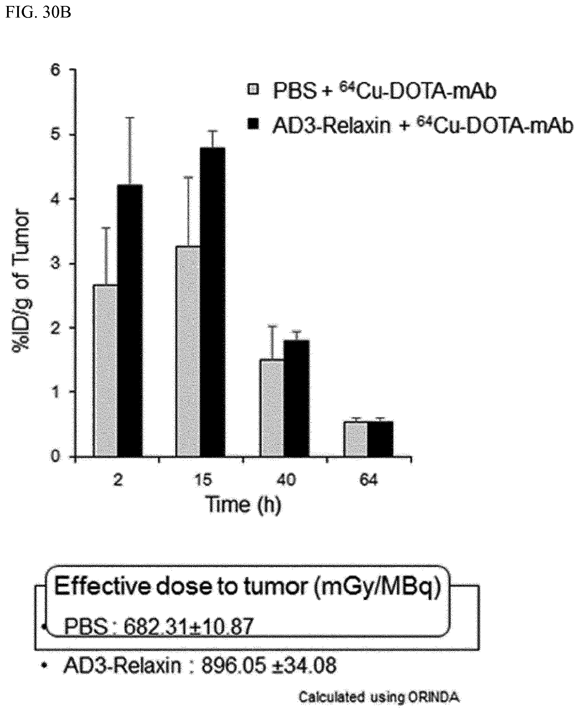

As illustrated in FIG. 27, it was confirmed that in the case of the tumor tissues to which PBS and trastuzumab were administered, trastuzumab was more intensively distributed around the periphery of the tumor tissue rather than the center of the tumor tissue, whereas when RdB/IL-12/RLX and trastuzumab were co-administered, trastuzumab was distributed uniformly throughout the tumor, and particularly, it could be seen that trastuzumab was diffused up to the center of the tumor tissue. Further, when the region marked in FIG. 27 was enlarged and observed, as illustrated in FIGS. 28A to 28C, in the case of the tumor tissue to which PBS and trastuzumab were administered, the infiltration of trastuzumab was increased as compared to the negative control, but as the region was further away from the margin site of the tumor tissue, the fluorescence of trastuzumab was reduced to the level of the negative control. However, it was confirmed that in the case of the tumor tissue to which RdB/IL-12/RLX and trastuzumab were co-administered, the infiltration of trastuzumab was maintained at high levels in not only the margin site, but also the center far away from the margin site. Furthermore, when the blood vessels of the tumor tissue were intensively enlarged and observed, as illustrated in FIGS. 29A to 29C, it was confirmed that in the case of the tumor tissue to which PBS and trastuzumab were administered, trastuzumab was increased only around the blood vessels, whereas in the case of the tumor tissue to which RdB/IL-12/RLX and trastuzumab were co-administered, trastuzumab was infiltrated into not only a region around the blood vessels, but also a region far away from the blood vessels. Finally, to check the time for trastuzumab to reside with a target in the tumor tissue, trastuzumab was labeled with ⁶⁴Cu-DOTA, and then PET imaging was performed. As a result, as illustrated in FIGS. 30A and 30B, it was confirmed that when RdB/IL-12/RLX (2.5 × 10¹⁰ VP) and trastuzumab were co-administered, trastuzumab distributed in vivo was concentrated in the tumor tissue while 15 hours began to elapse after the administration and trastuzumab still remained in the tumor tissue until 60 hours after the administration, as compared to the case where PBS and trastuzumab (150 µg/mice) were administered.

When these results are taken together, the inhibition of expression of extracellular matrix proteins in tumor tissues by RdB/IL-12/RLX may improve the anti-tumor effect by more enhancing the infiltration and diffusion effects of trastuzumab in tumors, so that RdB/IL-12/RLX is used in the form of administration in combination with preexisting anticancer agents and the like, thereby providing a stronger anti-tumor effect.

The above-described description of the present invention is provided for illustrative purposes, and the person skilled in the art to which the present invention pertains will understand that the present invention can be easily modified into other specific forms without changing the technical spirit or essential features of the present invention. Therefore, it should be understood that the above-described Examples are illustrative only in all aspects and are not restrictive.

## Claims

1. A pharmaceutical composition for aiding anticancer agents comprising a recombinant adenovirus comprising a gene encoding Interleukin 12 (IL-12); and a gene encoding decorin or relaxin.

2. The pharmaceutical composition of claim 1, wherein the recombinant adenovirus has one or more deletion regions selected from the group consisting of an E1 region and an E3 region.

3. The pharmaceutical composition of claim 2, wherein the gene encoding IL-12 is inserted into the E1 region of the recombinant adenovirus.

4. The pharmaceutical composition of claim 2, wherein the gene encoding decorin or relaxin is inserted into the E1 or E3 region of the recombinant adenovirus.

5. The pharmaceutical composition of claim 1, wherein the composition is administered simultaneously, separately, or sequentially in combination with immune checkpoint inhibitors.

6. The pharmaceutical composition of claim 5, wherein the immune checkpoint inhibitor is any one selected from the group consisting of programmed cell death-1 (PD-1) immune checkpoint inhibitors, programmed cell death-ligand 1 (PD-L1) immune checkpoint inhibitors, programmed cell death-ligand 2 (PD-L2) immune checkpoint inhibitors, cluster of differentiation 27 (CD27) immune checkpoint inhibitors, cluster of differentiation 28 (CD28) immune checkpoint inhibitors, cluster of differentiation 70 (CD70) immune checkpoint inhibitors, cluster of differentiation 80 (CD80, also known as B7-1) immune checkpoint inhibitors, cluster of differentiation 86 (CD86, also known as B7-2) immune checkpoint inhibitors, cluster of differentiation 137 (CD137) immune checkpoint inhibitors, cluster of differentiation 276 (CD276) immune checkpoint inhibitors, killer-cell immunoglobulin-like receptors (KIRs) immune checkpoint inhibitors, lymphocyte-activation gene 3 (LAG3) immune checkpoint inhibitors, tumor necrosis factor receptor superfamily immune checkpoint inhibitors, member 4 (TNFRSF4, also known as CD134) immune checkpoint inhibitors, glucocorticoid-induced TNFR-related protein (GITR) immune checkpoint inhibitors, glucocorticoid-induced TNFR-related protein ligand (GITRL) immune checkpoint inhibitors, 4-1BB ligand (4-1BBL) immune checkpoint inhibitors, cytolytic T lymphocyte associated antigen-4 (CTLA-4) antagonists, and a combination thereof.

7. The pharmaceutical composition of claim 1, wherein the cancer is any one selected from the group consisting of gastric cancer, lung cancer, non-small cell lung cancer, breast cancer, ovarian cancer, liver cancer, bronchial cancer, nasopharyngeal cancer, laryngeal cancer, pancreatic cancer, bladder cancer, colon cancer, cervical cancer, bone cancer, non-small cell bone cancer, hematologic malignancy, skin cancer, head or neck cancer, uterine cancer, colorectal cancer, anal near cancer, colon cancer, fallopian tube cancer, endometrial cancer, vaginal cancer, vulva cancer, Hodgkin's disease, esophageal cancer, small intestine cancer, endocrine cancer, thyroid cancer, parathyroid cancer, adrenal cancer, soft tissue sarcoma, urethral cancer, penile cancer, prostate cancer, chronic or acute leukemia, lymphocytic lymphoma, kidney or hydroureter cancer, renal cell carcinoma, renal pelvic carcinoma, salivary gland cancer, sarcoma cancer, pseudomyxoma peritonei, hepatoblastoma, testicular cancer, glioblastoma, cheilocarcinoma, ovarian germ cell tumors, basal cell carcinoma, multiple myeloma, gallbladder cancer, choroidal melanoma, cancer of the ampulla of Vater, peritoneal cancer, tongue cancer, small cell cancer, pediatric lymphoma, neuroblastoma, duodenal cancer, ureteral cancer, astrocytoma, meningioma, renal pelvis cancer, pudendum cancer, thymus cancer, central nervous system (CNS) tumors, primary central nervous system lymphoma, spinal cord tumors, brain stem neuroglioma, and pituitary adenoma.

8. The pharmaceutical composition of claim 1, wherein the cancer is a recurrent cancer or an anticancer agent-resistant cancer.

9. The pharmaceutical composition of claim 1, wherein the composition enhances anti-tumor immunity.

10. A method for enhancing the therapeutic efficacy of anticancer agents comprising: administering to an individual a recombinant adenovirus comprising a gene encoding Interleukin 12 (IL-12); and a gene encoding decorin or relaxin.

11. A method for treating cancer comprising: administering to an individual anticancer agents and a recombinant adenovirus comprising a gene encoding Interleukin 12 (IL-12); and a gene encoding decorin or relaxin.

12. A method for treating cancer comprising: administering to an individual immune checkpoint inhibitors and a recombinant adenovirus comprising a gene encoding Interleukin 12 (IL-12); and a gene encoding decorin or relaxin.
